# EUROPEAN PATENT APPLICATION

(11) **EP 3 510 867 A1**
(43) Date of publication of application: **17.07.2019**
(21) Application number: 18151443.1
(22) Date of filing: 12.01.2018
(51) Int. Cl.: A01N 43/50, A61K 8/49, A61K 8/84, A61L 2/18, A61K 31/4164, A61K 31/787, A01P 1/00, A01P 3/00

(54) **ANTIMICROBIAL POLYMER**

(71) Applicant: BASF SE, 67056 Ludwigshafen am Rhein (DE)
(72) Inventor: Lindner, Jean-Pierre Berkan, 67056 Ludwigshafen (DE); Kappert, Emiel Jan, 67056 Ludwigshafen (DE); Wiethan, Jürgen, 79639 Grenzach-Wyhlen (DE)
(74) Representative: BASF IP Association

(57) **Abstract**

Disclosed is a polymeric, ionic compound comprising imidazolium groups, which compound is obtainable by simultaneously reacting glyoxal, formaldehyde, at least one C₄-Csalkylene diamine, and at least one protic acid, especially by simultaneously reacting glyoxal, formaldehyde, hexylene diamine, and acetic acid in the presence of a polar solvent. The polymeric ionic compound is characterized in that its number average molecular weight is from the range 2500 to 19000 g/mol and its polydispersity is from the range 2 to 9. It shows advantageous antimicrobial properties, and may be used in various formulations for application fields including plant protection, personal care, home care, industrial or institutional or hospital disinfection, material protection, or pharmaceuticals.

## Description

The present invention relates to a new formulation with antimicrobial activity, to a new antimicrobial polymer, to the use of said formulation and said polymer as a biocide e.g. in formulations for cleaning, disinfection and/or descaling purposes, as well as to a cleaning agent or disinfection agent or descaling agent comprising said formulation.

US-5631274 proposes some imidazolium salts, including di-, tri- and tetramers thereof, as swimming pool sanitizers. WO2012127009 and WO2017025433 describe a number of imidazolium polymers with activity against Staphylococcus aureus and Pseudomonas aeruginosa.

It has now been found that a certain class of linear imidazolium polymers of defined molecular weight and limited distribution of chain lengths again shows distinctly improved effects.

The invention thus primarily pertains to an antimicrobial composition comprising at least one antimicrobial agent and at least one carrier and/or auxiliary agent, wherein an antimicrobial agent is a polymeric, ionic compound containing alkylene linked imidazolium groups.

While the present polymeric, ionic compound used as antimicrobial agent, as a polymerization product, consists of species of various chain lengths, it typically contains only low amounts of molecular species below 2000 g/mol; typically, species having a molar mass below 2000 g/mol make up less than 12 % of the total weight of the polymeric ionic compound, preferably less than 8 % b.w.

The polymeric, ionic compound is obtainable by reacting glyoxal, formaldehyde, at least one C4-C8alkylene diamine and at least one protic acid. It is characterized in that its number average molecular weight is from the range 2500 to 19000 g/mol, the fraction of species having a molar mass below 2000 g/mol is less than 12 % of the total weight of the polymeric ionic compound, and its polydispersity is from the range 2 to 9.

Polydispersity is the ratio Mw/Mn between the weight average molecular weight (Mw) and the number average of the molecular weight Mn. Preferred polymers typically show a polydispersity from the range 2.5 to 8.5. The number average molecular weight of preferred polymers is typically from the range 3000 to 19000, more preferably 4000 to 17000, especially 5000 to 10000 g/mol. In an especially preferred embodiment, the present polymeric, ionic compound further shows a weight average molecular weight (Mw) from the range 20000 to 80000, such as 25000 to 70000, or even 30000 to 60000 g/mol.

Molecular weight Mw, Mn and polydispersity are as determined by gel permeation chromatography, especially using modified polyacrylate-copolymer gel, poly(2-vinylpyridine) standards, and water containing 0.1% (w/w) trifluoroacetic acid and 0,1 mol of sodium chloride per liter as eluent at 35°C (see chromatographic method described further below).

The C4-C8alkylene diamine preferably is hexylene diamine; the reaction mixture for obtaining the polymeric, ionic compound generally does not contain any monoamine.

Typically, the polymeric, ionic compound in the present composition comprises repeating units of the formula 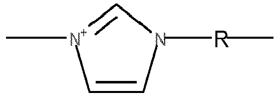 whose positive charges are counterbalanced by anions Ac⁻,
and preferably a polymer of the formula I 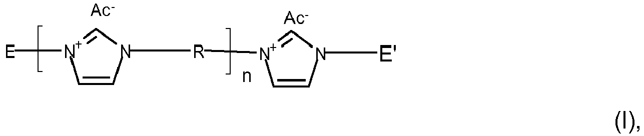 wherein
E is C4-C8 aminoalkyl or C4-C8 ammoniumalkyl, wherein preferably the alkyl is straight chain alkyl carrying a terminal amino or ammonium group;
E' is as defined for E, or is hydrogen; E' typically has the same meaning as E;
R is C4-C8alkylene, preferably of straight chain;
Ac- is the anion of the protonic acid used in the preparation method outlined above, such as an anion selected from HCOO, CH3COO, C₃-C₁₉ alkylcarboxylate, HSO₄, sulphate, nitrate, H₂PO₄, HPO₄, PO₄, C₁-C₈sulfonate, trifluormethyl-sulfonate, or halogenides such as chloride, bromide iodide; especially acetate; and
n denotes the number of repeating units to obtain a number average molecular weight from the range 2500 to 19000 g/mol. Thus, the present polymeric, ionic compound commonly contains typical molecular species wherein n ranges from 10 to 75, preferably from 15 to 60.

A preferred composition of the invention comprises a polymeric ionic compound, typically of the formula I, whose end groups, or E in formula I, is aminohexyl or ammoniumhexyl, and R is hexylene. Preferably, Ac- stands for the acetyl anion.

Thus, a preferred embodiment of the invention is an antimicrobial composition comprising, as an antimicrobial agent, at least one linear polymeric, ionic compound comprising imidazolium groups, and at least one carrier and/or at least one auxiliary agent, wherein the polymeric compound has a number average molecular weight from the range 3000 to 19000 g/mol and a polydispersity from the range 2 to 9, as described above, and preferably conforms to the above formula I such as exemplified by present polymers A, B, C and D, especially where a liquid carrier and at least one auxiliary from the groups (i), (ii), (iii) and (iv) described below is present.

Another preferred embodiment of the invention is an antimicrobial composition comprising, as an antimicrobial agent, at least one linear polymeric, ionic compound comprising imidazolium groups, and at least one carrier and/or at least one auxiliary agent, wherein the polymeric compound has a number average molecular weight from the range 4000 to 17000 g/mol and a polydispersity from the range 2 to 9, as described above, and preferably conforms to the above formula I such as exemplified by present polymers B, C and D, especially where a liquid carrier and at least one auxiliary from the groups (i), (ii), (iii) and (iv) described below is present.

Another preferred embodiment of the invention is an antimicrobial composition comprising, as an antimicrobial agent, at least one linear polymeric, ionic compound comprising imidazolium groups, and at least one carrier and/or at least one auxiliary agent, wherein the polymeric compound has a number average molecular weight from the range 5000 to 17000 and a polydispersity from the range 2 to 9, as described above, and preferably conforms to the above formula I such as exemplified by present polymers C and D, especially where a liquid carrier and at least one auxiliary from the groups (i), (ii), (iii) and (iv) described below is present.

An especially preferred embodiment of the invention is an antimicrobial composition comprising, as an antimicrobial agent, at least one linear polymeric, ionic compound comprising imidazolium groups, and at least one carrier and/or at least one auxiliary agent, wherein the polymeric compound has a number average molecular weight from the range 5000 to 10000 and a polydispersity from the range 2.5 to 8.5, as described above, and preferably conforms to the above formula I such as exemplified by present polymer C, especially where a liquid carrier and at least one auxiliary from the groups (i), (ii), (iii) and (iv) described below is present.

In this specification, the antimicrobial agent of the invention is synonymously recalled as "polymeric, ionic compound containing alkylene linked imidazolium groups", "polymeric, ionic compound comprising imidazolium groups", "polymeric, ionic compound", "linear imidazolium polymer", "imidazolium polymer", "polyimidazolium compound", "imidazolium compound", "antimicrobial polymer of the invention" or, with reference to the present preparation method, as "reaction product".

The composition of the present invention provides high antimicrobial activity, especially when applied in form of a formulation for cleaning, disinfection and/or descaling. The composition of the present invention further provides advantageous properties in the formulation, and in the application of biocidal formulations, such as reduced foam generation, better compatibility with auxiliary agents such as surfactants, stability of the liquid formulation, and good dosability. Advantageous carriers and/or auxiliary agents, especially for these purposes, are as noted below. The composition of the present invention further has the advantage of safer application with respect to toxic effects like undesired skin sensitization, which may be avoided when using the present composition.

### Preparation

Present linear imidazolium polymer is advantageously prepared in analogy to methods described in WO 2016/020214, WO 2012/127009, WO 2010/072571, and literature cited therein, by reaction of glyoxal (i.e. ethane-1,2-dione), formaldehyde and alkylene diamine in presence of a protic acid and a solvent. Typically, educts are used in about equimolar amounts, and the protic acid is used in an amount about equal with the moles of amine moieties in the reaction mixture.

The term "about" in the context of the present invention denote an interval of accuracy that the person skilled in the art will understand to still ensure the technical effect of the feature in question. The term typically indicates deviation from the indicated numerical value of ±20 %, preferably ±15 %, more preferably ±10 %, and even more preferably ±5 %, except in case of temperatures, where the term "about" denotes a possible deviation by plus or minus 5 degrees (preferably up to plus or minus 2.5 degrees). The term "essentially" is synonymous to "about" and denotes a possible deviation from the basic value as noted above.

The protic acid in the reaction mixture for obtaining the polymeric, ionic compound is preferably selected from C1-C20carboxylic acids and mineralic acids such as formic acid, hydrochloric acid, hydrobromic acid, acetic acid, aliphatic carboxylic acids having 3 to 20 carbon atoms, H2SO4, HNO3, H3PO4, C1-C8sulfonic acids, trifluormethyl-sulfonic acid; especially preferred is acetic acid.

Methods for obtaining the present linear imidazolium polymer will be described below in more detail.

The reaction of present educts glyoxal, formaldehyde and alkylene diamine is carried out simultaneously. The reaction may be carried out by mixing the reactands, e.g. by adding the reactands to the protic acid, in the presence of a suitable solvent. Typically, a polar solvent (different from a protic acid) is used such as water or mixtures of water with other protic solvents such as an alcohol. Some protic acids such as carboxylic acid may also be used as additional solvent by using an excess of such acid. Typically, protic acid or its solution may be furnished in a suitable reaction vessel, advantageously with stirring, and solutions of the aldehydes and the diamine are simultaneously added while controlling the temperature of the mixture. Advantageously, the alkylene diamine and the aldehyde solution containing glyoxal and formaldehyde are added to the protic acid separately, e.g. using separate feeds. In a preferred process, the molar ratio of each of the aldehydes to the diamine in the reaction mixture is not lower than 1 during each time period of the addition step, the ratio preferably being from the range 1 to 1.2, especially 1 to 1.05. Temperature of the mixture is preferably controlled during this step to remain in the lower liquid range, such as -20°C to about 40°C, or 0 to 30°C. Pressure is only of minor importance, e.g. to ensure a certain liquid range such as about -5° to about 100°C in case of atmospheric pressure and use of water as the solvent. After addition of all components, the reaction mixture advantageously is heated e.g. to about 60 to 150°C, or up to the boiling point, for a time period of about 0.1 to 10 hours. A convenient method uses a temperature from about 0° C to about 100° C throughout the reaction. In another preferred reaction, temperature of the mixture is kept between about 0° C and about 50° C with cooling, typically between about 15 and 30° C, during the addition step; subsequently, temperature may be raised to a temperature between about 50° to the boiling point of the solvent or solvent mixture at normal pressure, e.g. to about 80 to 105° C. Depending on the amounts of educts used and the cooling conditions, the addition of educts may be completed within a time period ranging from about 5 minutes up to many hours, e.g. 0.5 to about 10 hours. Similarly, the optional subsequent heating step may last from about 5 minutes up to many hours, e.g. 15 minutes to about 5 hours.

After completion of the reaction, the mixture comprising the polymer of the invention typically has a pH from the acidic range, e.g. 3-7, especially 5-6 in case that acetic acid is used as the protic acid. The mixture may be used as such, or may be subjected to further derivatization (e.g. reacting with alkylating agent) and/or purification steps (e.g. filtration as described below, removal of the solvent under reduced pressure and/or heating to the boiling point).

Present linear imidazolium polymer is prepared in the presence of a suitable solvent, typically a polar solvent compatible with the educts and end product. Preferred solvents are water, alcohols like C1-C4alcohols, esters, amides, and mixtures of such solvents, especially water and C1-C4alcohols. Typically, the educts formaldehyde and glyoxal are added to the reaction mixture as a solution in such solvent.

Obtaining the desired molecular weight and distribution of chain lengths may be facilitated by an additional step of ultrafiltration or dialysis. Thus, the crude product obtained from the polymerization reaction may be subjected to ultrafiltration, e.g. by filling the diluted solution of the product into a filtration cell fitted with suitable membrane with cut-off e.g. between 5 and 15 kD molecular weight, and pressurizing the cell.

The imidazolium polymer thus obtained may be subjected to an anion exchange. This allows the preparation of imidazolium polymers even with anions for which no corresponding stable protic acid exists. The anion exchange can be effected by known methods, e.g. transprotonation, reaction with a metal salt, ion exchange chromatography, electrolytically or by means of a combination of these measures. Anions of the present polymer (expressed as Ac⁻ in the below formula I) thus may comprise any convenient anion, such as halide, sulfate, hydrogensulfate, alkylsulfate, arylsulfate, perfluoro alkyl- and arylsulfates, sulfonate, alkylsulfonate, arylsulfonate, perfluoro alkyl- and arylsulfonates, phosphate, halophosphate, alkylphosphate, nitrate, perchlorate, tetrachloroaluminate, tetrafluoroborate, alkylborate, saccharinate, alkyl carboxylates or bis(perflouroalkylsulfonyl)anions. Anions also comprise halogenides and pseudo-halogenides such as F⁻, Cl⁻, Br⁻, I⁻, CN⁻, SCN⁻, OCN⁻, NO₂⁻, NO₃⁻, N(CN)⁻, or BF₄⁻, PF₆⁻, AlF₄⁻, ALCL₄⁻, AlBr₄⁻, ZnCl₃⁻, SnCl₄⁻, CuCl₂⁻; the group of sulfates, sulfites and sulfonates, the group of phosphates; the group of phosphonates and phosphinates; the group of phosphites; the group of phosphonites and phosphinites; the group of carboxylates and polybasic carboxylic acids; the group of boronates; the group of silicates and silicic esters; the group of alkylsilane and arylsilane salts; the group of carboximides, bis(sulfonyl)imides and sulfonylimides.

The molecular weight of the antimicrobial polymer of the invention thus obtained typically is from a range as noted further above. Preferred is an antimicrobial polymer of the invention showing a polydispersity (Mw/Mn) from the range 2.5 to 8.5. The invention thus includes products comprising varying chain length, such as polymeric ionic compounds obtainable as described above and characterized by a molecular weight as noted hereinabove and showing a polydispersity (Mw/Mn) from the range noted above. The present composition thus comprises more than one individual linear polyimidazolium compound; typically, the present composition thus comprises a mixture of polymeric compounds each of which falling under formula I.

### Formulations

The invention thus primarily pertains to an antimicrobial composition comprising, as an antimicrobial agent, at least one linear polymeric, ionic compound comprising imidazolium groups, and at least one carrier and/or at least one auxiliary agent, wherein the polymeric compound has a number average molecular weight from the range 2500 to 19000 g/mol and a polydispersity from the range 2 to 9, is obtainable by a method as described above, and preferably conforms to the above formula I.

The antimicrobial composition according to the invention can be provided and/or applied as a solid or as a liquid. This encompasses compositions in form of aerosols. The biocide composition according to the invention can be formulated e.g. as powder, granulate, pellets, pills, agglomerates, solutions, emulsions, suspensions, dispersions, pastes, in combination with carrier materials, etc. Preferred are liquid formulations comprising at least one carrier, which is liquid under standard conditions.

Generally, the antimicrobial compositions according to the invention can be formulated free from solvent or with a suitable solvent as a carrier. Generally, the imidazolium compounds used according to the invention are soluble in most protic solvents, swellable in most aprotic polar solvents and insoluble in most nonpolar solvents. Solvents suitable as carriers for the biocide compositions according to the invention are selected from among water, alcohols, such as methanol, ethanol, n-propanol, isopropanol, n-butanol, tert-butanol, diols and polyols, such as ethanediol and propanediol, amino alcohols, such as ethanolamine, diethanolamine and triethanolamine, ethers, e.g. tetrahydrofuran, diethyl ether, methyl tert-butyl ether and diethylene glycol monomethyl ether, ketones, such as acetone and methyl ethyl ketone, esters, e.g. ethyl acetate, formamide, dimethylformamide (DMF), dimethylacetamide, dimethyl sulfoxide (DMSO), acetonitrile, and mixtures thereof. In certain cases, also aromatic solvents, e.g. benzene, toluene, ethylbenzene or xylenes, halogenated solvents, e.g. dichloromethane, chloroform, carbon tetrachloride, dichloroethane or chlorobenzene, aliphatic solvents, e.g. pentane, hexane, heptane, octane, ligroin, petroleum ether, cyclohexane and decalin, and mixtures thereof or with the above solvents, may be used. The solvent is preferably selected from among water, water-miscible organic solvents and mixtures thereof. The solvent is particularly preferably selected from among water, methanol, ethanol, n-propanol, isopropanol, n-butanol, tert-butanol and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

A multitude of different auxiliaries such as active substances and additives can be formulated in the biocide compositions according to the invention. Suitable auxiliaries include surfactants, thickeners, tackifiers and binders, chelating agents, further antimicrobial agents such as bactericides and fungicides, dispersants, emulsifiers, wetting agents, adjuvants, solubilizers, penetration enhancers, protective colloids, adhesion agents, humectants, repellents, attractants, feeding stimulants, compatibilizers, anti-freezing agents, anti-foaming agents, perfumes, colorants. In some cases auxilliaries especially perfumes can also be formulated at least partly in encapsulated form in order to enhance stability of the perfume or other auxiliaries and/or to facilitate the manufacturing process of the formulation. A detailed description of the encapsulation technologies is described by Unilever PLC in patent application EP 2838982.

The composition of the invention preferably comprises a liquid carrier and at least one auxiliary agent. The liquid carrier preferably comprises a solvent, such as water and/or one or more alcohols. In one preferred embodiment, the auxiliary agent comprises a surfactant.

Suitable surfactants are surface-active compounds, such as cationic, nonionic and amphoteric surfactants, block polymers, polyelectrolytes, and mixtures thereof. In certain cases, anionic surfactants may be applied as well. Covered are also combinations of various surfactant types (cationic, nonionic, amphoteric and anionic surfactants) in order to optimize the applicatory features e.g. cleaning properties and antimicrobial activity of the formulation.

The surfactants may be used in liquid form or in solid forms such as powders, granules, compacts, flakes or pastes.

Such surfactants can be used as emusifier, dispersant, solubilizer, wetter, penetration enhancer, protective colloid, or adjuvant. They are for instance described in the disclosure IP.com Journal (2011), 12(1A), IPCOM000213522D, published on 20th December 2011 in section (b) and in IP.com Journal (2016), IP246396D published on 3rd June 2016. Examples of surfactants are listed in McCutcheon's, Vol.1 : Emulsifiers & Detergents, McCutcheon's Directories, Glen Rock, USA, 2008 (International Ed. or North American Ed.).

Among surfactants, nonionic surfactants are most preferable as components of the present composition.

Such nonionic surfactants are typically selected from:
a) Alcohol ethoxylates such as C8-C18 fatty alcohols or oxo alcohols or Guerbet alcohols, each containing about 2 to 100 ethoxy units (commercially available e.g. from BASF SE under the trade name Dehydol LS, Dehydol LT, Dehydol 100, Lutensol AT, Lutensol AO, Lutensol M, Lutensol TO, Lutensol XA, Lutensol XP, Lutensol XL, Lutensol ON, Dehydol 04 Deo, Dehydol O4, Emulan HE 50, Lutensol PO 5, Lutensol CS 6250, Degressal SD, Dehydem Supra, Dehypon G 162, Dehypon OCP 502, Dehypon 2574, Dehypon LS, Plurafac LF, Plurafac SLF, Dehypon LT, Dehypon O, Dehypon G, Dehypon E, Dehypon GRA, Dehypon KE, Dehypon WET, Dehydrol 980);
b) Alkylated polyglycosides (also known as "sugar surfactant") comprising a carbohydrate as hydrophilic moiety and fatty alcohols as hydrophobic component, which may be represented by the general formula:

   **R-O-Gₚ**

   where R is alkyl or alkenyl (especially C8-C14),
   G is the residue of an aldose or ketose and
   P is 1, 2, 3, 4, 5, 6, 7, 8, 9 or 10; examples of such surfactants, and their combinations with antimicrobial agents, which may also be used within the present invention, are disclosed in WO 2013/045340; in a further variant, the sugar surfactants to be used according to the invention are alkyl and/or alkenyl polyglycosides of the above formula in which R is an alkyl and/or alkenyl radical having 4 to 22 carbon atoms, G is a sugar radical having 5 or 6 carbon atoms and P is numbers from 1 to 10. In a further embodiment, the alkyl and/or alkenyl polyglycosides are derived from aldoses or ketoses having 5 or 6 carbon atoms; the component G in the above formula is selected in one embodiment from the group of hexoses, preferably from the group comprising allose, altrose, glucose, mannose, gulose, idose, galactose, talose, psicose, fructose, sorbose, tagatose, particularly preferably glucose, or the component G in the above formula is selected from the group of pentoses, preferably the group comprising ribulose, xylulose, ribose, arabinose, xylose, lyxose, particularly preferably xylose and/or arabinose; such nonionic surfactants are commercially available e.g. from BASF SE under the trade names Dehypound W 07 N, Glucopon 215 UP, Glucopon 225 DK, Glucopon 425 N/HH, Glucopon GD 70, Glucopon 50 G, Glucopon 600 CSUP, Glucopon 650 EC, Plantatex LLE;
c) Amine ethoxylates, such as C10-C18 fatty amines ethoxylated with 5 to 50 ethoxy units, and blends of such amine ethoxylates with ethoxylated alcohols noted above (commercially available e.g. from BASF SE under the trade names Lutensol FA 12K, Demelan VPC);
   and/or
d) Polyethers such as polyethylene glycol, polypropylene glycol, and copolymers comprising polyethylene and polypropylene (typically block copolymers) such as available from BASF SE under the trade names Pluronic PE, Pluronic RPE, Pluriol E, Pluriol P.

As noted above, typical non-ionic surfactants are condensated products of ethylene oxide and/or propylene oxide with various reactive hydrogen-containing compounds reactive therewith having long hydrophobic chains (e.g. aliphatic chains of about 8 to 20 carbon atoms), which condensation products ("ethoxamers") contain hydrophilic polyoxyethylene moieties, such as condensation products of poly(ethyleneoxide) with fatty acids, fatty alcohols, fatty amides, polyhydric alcohols (e.g. sorbitan monostearate) and polypropylene oxide. Polyoxamers are e.g. block copolymers of polyoxyethylene and polyoxypropylene having an average molecular weight from about 3000 to 5000 and a preferred average molecular weight from about 3500 to 4000 and containing about 10-80% hydrophilic polyoxyethylene groups, by weight, of the block copolymer.

Further useful surfactants are amphoteric surfactants like C8-C18-betains, C8-C18-sulfobetains, C8-C24-alkylamido-C1-C4-alkylene betaines, imidazoline carboxylates, alkylamphocarboxycarbonic acids, alkylamphocarbonic acid (e.g. lauroamphoglycinate) and N-alkyl-beta-aminopropionate or-iminodipropionate can be used. In particular, the C10-C20-alkylamido-C1-C4-alkylenbetaine and coco fatty acid amide propylbetaine.

Further examples of non-ionic surfactants include the zwitterionic and amphoteric surfactants of US Pat. 2,658,072, 2,438,091, and 2,528,378, which are incorporated herein by reference.

In another preferred embodiment, the auxiliary agent comprises a polymer or thickener, often in addition to the above surfactant. Such components are typically selected from polyethylene imines (e.g. available under the trade names Lupasol PN 60 or Sokolan HP 20), cationic polyacrylates or polyacrylamides (e.g. available under the trade names Polyquart 149, Rheovis CDE, Rheovis FRC, Rheovis CSP), nonionic or amphoteric polymers or polyvinylpyrrolidones (e.g. available under the trade names Polyquart Pro A, Polyquart Ecoclean, Sokolan HP 22 G, Sokalan HP 50, Sokalan HP 53, Sokalan HP 165, Sokalan HP 56, Sokalan HP 66 K).

In another preferred embodiment, the auxiliary agent comprises a chelating agent, especially in addition to the above surfactant. Such chelating agents are typically selected from polycarboxylic acids and salts thereof, such as ethylenediaminetetraacetic acid (EDTA), the disodium salt or tetrasodium salt of EDTA, the trisodium salts of methylglycinediacetic acid (MGDA) or nitrilotriacetic acid (NTA), which components are commercially available from BASF SE under the trade names Trilon M, Trilon A, Trilon B, Trilon BD, Trilon BS, Trilon BX. The chelating agents used are available in various forms such as liquids, powders, compacts, granules.

In another preferred embodiment, the auxiliary agent comprises a further antimicrobial agent, especially in addition to the above surfactant. Such agents are typically selected from aldehydes, alcohols and chlorinated hydroxydiphenylethers, examples such as glutaraldehyde, glyoxal, phenoxyethanol, triclosan (the compound 2,4,4'-trichloro-2'-hydroxydiphenylether), and diclosan (i.e. the compound 4,4'-dichloro-2-hydroxydiphenylether) are commercially available from BASF SE under the trade names Protectol GA, Protectol GL, Protectol PE, Tinosan HP 100.

A preferred embodiment of the invention thus is an antimicrobial composition comprising, as an antimicrobial agent, at least one linear polymeric, ionic compound comprising imidazolium groups, and at least one carrier and/or at least one auxiliary agent, wherein the polymeric compound has a molecular weight and polydispersity as defined further above, is obtainable by a method as described above, and preferably conforms to the above formula I, wherein the auxiliary agent is selected from
i) non-ionic surfactants, such as polyethers or condensated products of ethylene oxide and/or propylene oxide with fatty acids, fatty alcohols, fatty amides, polyhydric alcohols; especially alcohol ethoxylates, alkylated polyglycosides, amine ethoxylates, polyethers;
ii) thickeners and binders, such as polyethylene imines, cationic polyacrylates, cationic polyacrylamides, polyvinylpyrrolidones, and nonionic or amphoteric polymers;
iii) chelating agents like polycarboxylic acids and salts thereof, such as ethylenediaminetetraacetic acid, its di-, tri- or tetrasodium salt, the trisodium salt of methylglycinediacetic acid, nitrilotriacetic acid and salts thereof;
iv) further antimicrobial agents, especially aldehydes, alcohols, chlorinated hydroxydiphenylethers, such as glutaraldehyde, glyoxal, phenoxyethanol, triclosan, diclosan.

Especially preferred is a composition as noted above containing an auxiliary agent selected from the groups (i), (ii), (iii) and (iv), and a liquid carrier such as water, water-miscible organic solvents like methanol, ethanol, n-propanol, isopropanol, n-butanol, tert-butanol, and mixtures thereof. Of special technical importance is such composition comprising the liquid carrier and a surfactant (especially from the above group (i)) and a further component of group (ii). Also of special technical importance is such composition comprising the liquid carrier and a surfactant (especially from the above group (i)) and a further component of group (iii). Also of special technical importance is such composition comprising the liquid carrier and a surfactant (especially from the above group (i)) and a further component of group (iv).

The composition of the invention contains the polymeric, ionic compound comprising imidazolium groups generally in an amount ranging from 0.0001 to 30 % by weight of the composition; specific dosages for typical applications are given further below. Liquid concentrates (typically aqueous and or alcoholic, e.g. using a polyhydric alcohol such as propylene glycol as the solvent) may be formulated, which contain up to 70% by weight of the present imidazolium polymer, typically 10 to 70%; an example is an aqueous solution containing about 50% by weight of the polymer. The auxiliary agent, too, is often present in an amount ranging from 0.0001 to 30 % by weight of the composition. In case that the present composition comprises a surfactant as the auxiliary (or one of the auxiliaries), this may be the main component; in case of a solid composition (e.g. a powder composition), the surfactant may make up e.g. 10 to 95% by weight of the composition. In case that the present composition is a liquid composition, a liquid carrier is typically present as a main component, making up e.g. 30 to 99.9999 % b.w. of the total composition, or, if an auxiliary is present, e.g. 30 to 98 % b.w. of the composition. The amounts of the present polymeric, ionic compound comprising imidazolium groups, of the carrier and of the auxiliary generally add up to 100 % of the composition.

The formulation of the invention may comprise the polymeric imidazolium compound of the invention in the mixture as obtained, e.g. of pH from the range 5 to 6, or in a pH adjusted way (typically after addition of a suitable base such as NaOH or KOH, or after addition of a suitable acid such as acetic acid or methanesulfonic acid in case that a low pH is desired). Typical is an aqueous formulation of a pH from the neutral to acidic range, e.g. from the range of pH 3 to 7, e.g. 5 to 6, or an aqueous formulation, which has a pH from the range of 7 to 10; the aqueous formulation contains at least 50 wt.-%, especially 80 to 99.8 wt.-%, of water.

Suitable adjuvants are compounds which have a neglectable or even no bioicidal activity themselves, and which improve the biological performance of the polymeric, ionic compound comprising imidazolium groups on the target. Examples are surfactants, mineral or vegetable oils, and other auxilaries. Further examples are listed by Knowles,

Adjuvants and additives, Agrow Reports DS256, T&F Informa UK, 2006, chapter 5.

Further suitable thickeners are polysaccharides (e.g. xanthan gum, carboxymethylcellulose), anorganic clays (organically modified or unmodified), polycarboxylates, and silicates. Further suitable bactericides and antimicrobial agents, such as bronopol and isothiazolinone derivatives, are as listed below.

Suitable anti-freezing agents are ethylene glycol, propylene glycol, urea and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Further suitable tackifiers or binders are polyvinylpyrrolidons, polyvinylacetates, polyvinyl alcohols, polyacrylates, biological or synthetic waxes, and cellulose ethers

Suitable surfactants such as cationic, amphoteric, non-ionic and anionic surfactants are widely known in the art.

Suitable cationic surfactants include quaternary surfactants, for example quaternary arrnmonium compounds with one or two hydrophobic groups, or salts of long-chain primary amines. Examples of suitable cationic surfactants are quaternized ethoxylated fatty amines, quaternized ethoxylated fatty oils and blends thereof and with further surfactants such as Dehyquart CSP, Demelan AU-39/Bio (commercially available from BASF SE). Preference is given to ammonium halides, such as alkyltrimethylammonium chlorides, dialkyldimethylammonium chlorides and trialkylmethylammonium chlorides, for example cetyltrimethylammonium chloride, stearyltrimethylammonium chloride, distearyldimethy-lammonium chloride, lauryldimethylammonium chloride, lauryldimethylbenzylammonium chloride and tricetylmethylammonium chloride. Further cationic surfactants that can be used in accordance with the invention are quaternised protein hydrolysates.
Also suitable in accordance with the invention are cationic silicone oils, such as, for example, the commercially available products Q2-7224 (manufacturer: Dow Corning; a stabilised trimethylsilylamodimethicone), Dow Corning 929 emulsion (comprising a hydroxylamino-modified silicone, which is also referred to as amodimethicone), SM-2059 (manufacturer: General Electric), SLM-55067 (manufacturer: Wacker) and also Abil®-Quat 3270 and 3272 (manufacturer: Th. Goldschmidt; diquaternary polydimethylsiloxanes, quaternium-80), or silicones, as described in WO 00/12057, especially page 45, line 9 to page 55, line 2.
Alkylamidoamines, especially fatty acid amidoamines, such as the stearylamidopropyldimethylamine obtainable under the name Tego Amid® 18, are distinguished not only by a good conditioning action but also especially by their good biodegradability. Quaternary ester compounds, so-called "esterquats", such as the methyl hydroxyalkyldialkoyloxyalkylammonium methosulfates marketed under the trademark Stepantex®, are also very readily biodegradable.

An example of a quaternary sugar derivative that can be used as cationic surfactant is the commercial product Glucquat®100, according to CTFA nomenclature a "lauryl methyl gluceth-10 hydroxypropyl dimonium chloride".

A wide variety of anionic surfactants are potentially useful herein. Several examples of suitable anionic surfactants are disclosed in US Pat 3,929,678, which is incorporated herein by reference.

Although anionic surfactants are not preferred for formulations with cationic biocides such as the present polymeric ionic compound, formulations of the invention may contain anionic surfactants, e.g. in order to adjust certain properties of the formulation. Examples of anionic surfactants include those selected from the group consisting of alkyl and alkyl ether sulfates, sulfated monoglycerides, sulfonated olefins, alkyl aryl sulfonates, primary or secondary alkane sulfonates, alkyl sulfosuccinates, acyl taurates, acyl isethionates, alkyl glycerylether sulfonate, sulfonated methyl esters, sulfonated fatty acids, alkyl phosphates, acyl glutamates, acyl sarcosinates, alkyl sulfoacetates, acylated peptides, alkyl ether carboxylates, acyl lactylates, anionic fluorosurfactants, and mixtures thereof. Mixtures of anionic surfactants can be used effectively in the present invention.

Alkyl and alkyl ether sulfates have the respective formulae R₁-O-SO₃-M and R₁-(CH₂H₄-O)ₓ-O-SO₃-M, wherein R₁ is a saturated or unsaturated, branched or unbranched alkyl group from about 8 to about 24 carbon atoms, x is 1 to 10, and M is a water-soluble cation such as ammonium, sodium, potassium, magnesium, triethanolamine, diethanolamine and monoethanolamine. The alkyl sulfates are typically made by the sulfation of monohydric alcohols (having from about 8 to about 24 carbon atoms) using sulfur trioxide or other known sulfation technique. The alkyl ether sulfates are typically made as condensation products of ethylene oxide and monohydric alcohols (having from about 8 to about 24 carbon atoms) and then sulfated. These alcohols can be derived from fats, e.g., coconut oil or tallow, or can be synthetic. Common examples of alkyl sulfates include sodium, ammonium, potassium, magnesium, or TEA salts of lauryl or myristyl sulfate. Common examples alkyl ether sulfates include ammonium, sodium, magnesium, or TEA laureth-3 sulfate.

Another class of anionic surfactants potentially useful are sulfated monoglycerides of the formula R₁-CO-O-CH₂-C(OH)H-CH₂-O-SO₃-M, wherein R₁ is a saturated or unsaturated, branched or unbranched alkyl group from about 8 to about 24 carbon atoms, and M is a water-soluble cation such as ammonium, sodium, potassium, magnesium, triethanolamine, diethanolamine and monoethanolamine. These are typically made by the reaction of glycerin with fatty acids (having from about 8 to about 24 carbon atoms) to form a monoglyceride and the subsequent sulfation of this monoglyceride with sulfur trioxide. An example of a sulfated monoglyceride is sodium cocomonoglyceride sulfate.

Other anionic surfactants potentially useful include olefin sulfonates of the form R₁SO₃-M, wherein R₁ is a mono-olefin having from about 12 to about 24 carbon atoms, and M is a water-soluble cation such as ammonium, sodium, potassium, magnesium, triethanolamine, diethanolamine and monoethanolamine. These compounds can be produced by the sulfonation of D-olefins by means of uncomplexed sulfur trioxide, followed by neutralization of the acid reaction mixture in conditions such that any sultones which have been formed in the reaction are hydrolyzed to give the corresponding hydroxyalkanesulfonate. An example of a sulfonated olefin is sodium C₁₄/C₁₆ D-olefin sulfonate.

Other anionic surfactants potentially useful are the linear alkylbenzene sulfonates of the form R₁-C₆H₄-SO₃-M, wherein R₁ is a saturated or unsaturated, branched or unbranched alkyl group from about 8 to about 24 carbon atoms, and M is a water-soluble cation such as ammonium, sodium, potassium, magnesium, triethanolamine, diethanolamine and monoethanolamine. These are formed by the sulfonation of linear alkyl benzene with sulfur trioxide. An example of this anionic surfactant is sodium dodecylbenzene sulfonate.

Still other anionic surfactants include the primary or secondary alkane sulfonates of the form R₁-SO₃-M, wherein R₁ is a saturated or unsaturated, branched or unbranched alkyl chain from about 8 to about 24 carbon atoms, and M is a water-soluble cation such as ammonium, sodium, potassium, magnesium, triethanolamine, diethanolamine and monoethanolamine. These are commonly formed by the sulfonation of paraffins using sulfur dioxide in the presence of chlorine and ultraviolet light or another known sulfonation method. The sulfonation can occur in either the secondary or primary positions of the alkyl chain. An example of an alkane sulfonate useful herein is alkali metal or ammonium C13-C17 paraffin sulfonates.

Still other anionic surfactants potentially useful are the alkyl sulfosuccinates, which include disodium N-octadecylsulfosuccinamate; diammonium lauryl sulfosuccinate; tetrasodium N-(1,2-dicarboxyethyl)-N-octadecylsulfosuccinate; diamyl ester of sodium sulfosuccinic acid; dihexyl ester of sodium sulfosuccinic acid; and dioctyl esters of sodium sulfosuccinic acid.

Also useful may be taurates which are based on taurine, also known as 2-aminoethanesulfonic acid. Examples of taurates include N-alkyltaurines such as the one prepared by reacting dodecylamine with sodium isethionate according to the teaching of US Pat 2,658,072 which is incorporated herein by reference in its entirety. Other examples based of taurine include the acyl taurines formed by the reaction of n-methyl taurine with fatty acids (having from about 8 to about 24 carbon atoms).

Another class of anionic surfactants potentially useful are the acyl isethionates. The acyl isethionates typically have the formula R₁-CO-O-CH₂-CH₂SO₃-M, wherein R₁ is a saturated or unsaturated, branched or unbranched alkyl group having from about 10 to about 30 carbon atoms, and M is a cation. These are typically formed by the reaction of fatty acids (having from about 8 to about 30 carbon atoms) with an alkali metal isethionate. Non-limiting examples of these acyl isethionates include ammonium cocoyl isethionate, sodium cocoyl isethionate, sodium lauroyl isethionate, and mixtures thereof.

Still other anionic surfactants potentially useful are the alkylglyceryl ether sulfonates of the form R₁-OCH₂-C(OH)H-CH₂-SO₃-M, wherein R₁ is a saturated or unsaturated, branched or unbranched alkyl group from about 8 to about 24 carbon atoms, and M is a water-soluble cation such as ammonium, sodium, potassium, magnesium, triethanolamine, diethanolamine and monoethanolamine. These can be formed by the reaction of epichlorohydrin and sodium bisulfite with fatty alcohols (having from about 8 to about 24 carbon atoms) or other known methods. One example is sodium cocoglyceryl ether sulfonate.

Other anionic surfactants potentially useful include the sulfonated fatty acids of the form R₁-CH(SO₄)-COOH and sulfonated methyl esters of the from R₁-CH(SO₄)-CO-O-CH₃, where R₁ is a saturated or unsaturated, branched or unbranched alkyl group from about 8 to about 24 carbon atoms. These can be formed by the sulfonation of fatty acids or alkyl methyl esters (having from about 8 to about 24 carbon atoms) with sulphur trioxide or by another known sulfonation technique. Examples include alpha sulphonated coconut fatty acid and lauryl methyl ester.

Other anionic materials potentially useful as surfactants include alkyl ether carboxylates corresponding to the formula R₁-(OCH₂CH₂)_{X}-OCH₂-CO₂-M wherein R₁ is a saturated or unsaturated, branched or unbranched alkyl or alkenyl group of about 8 to about 24 carbon atoms, x is 1 to 10, and M is a water-soluble cation. Nonlimiting examples of which include sodium laureth carboxylate.

Other anionic materials potentially useful as surfactants include acyl lactylates corresponding to the formula R₁-CO-[O-CH(CH₃)-CO]_{X}-CO₂-M wherein R₁ is a saturated or unsaturated, branched or unbranched alkyl or alkenyl group of about 8 to about 24 carbon atoms, x is 3, and M is a water-soluble cation, non-limiting examples of which include sodium cocoyl lactylate.

Other anionic materials potentially useful as surfactants include the carboxylates, non-limiting examples of which include sodium lauroyl carboxylate, sodium cocoyl carboxylate, and ammonium lauroyl carboxylate. Anionic flourosurfactants can also be used.

Other anionic materials include acyl glutamates corresponding to the formula R₁-CO-N(COOH)-CH₂CH₂-CO₂-M wherein R₁ is a saturated or unsaturated, branched or unbranched alkyl or alkenyl group of about 8 to about 24 carbon atoms, and M is a water-soluble cation. Nonlimiting examples of which include sodium lauroyl glutamate and sodium cocoyl glutamate.

Other anionic materials include alkanoyl sarcosinates corresponding to the formula R₁-CON(CH₃)-CH₂CH₂-CO₂-M wherein R₁ is a saturated or unsaturated, branched or unbranched alkyl or alkenyl group of about 10 to about 20 carbon atoms, and M is a water-soluble cation. Nonlimiting examples of which include sodium lauroyl sarcosinate, sodium cocoyl sarcosinate, and ammonium lauroyl sarcosinate.

Any counter cation, M, can be used on the anionic surfactant. Typically the counter cation is selected from the group consisting of sodium, potassium, ammonium, monoethanolamine, diethanolamine, and triethanolamine.

The formulation of the invention typically contains a surfactant, such as a cationic, an anionic, amphoteric or preferably a non-ionic surfactant; mixtures of cationic, anionic. amphoteric and/or non-ionic surfactants such as mixtures of anionic and non-ionic surfactants, or mixtures of cationic and non-ionic surfactants may also be used.

The imidazolium polymers used in accordance with the invention, as well as formulations of the invention containing such polymers, exhibit pronounced antimicrobial action, for example against pathogenic gram-positive bacteria, gram-negative bacteria, mycoplasma, yeasts and molds and viruses (including enveloped and non-enveloped viruses). They are effective against fungi and bacteria and, most importantly, they retain their effectiveness against fungi and bacteria even when in in combination with non-ionic surfactants. They further show pronounced anti-biofilm activity, preventing or inhibiting the formation of a bio-film, with disruption, kill and/or removal of an existing biofilm, which allows for the preparation of efficient formulations for clean-in-place (CIP), water treatment or sanitary disinfection, as described further below.

The formulation of the invention may generally contain further antimicrobial agents, such as
a chloro benzene derivative of the formula (II) 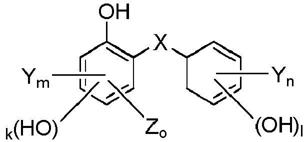 wherein
   X is O, S or -CH2-
   Y is Cl or Br,
   Z is SO2H, NO2 or C1 to C4 alkyl,
   k is 0 or 1,
   I is 0 or 1,
   m is 0, 1, 2, or 3,
   n is 0, 1, 2, or 3,
   o is 0 or 1,
   which preferably is triclosan or especially diclosan (i.e. the compound 4,4'-dichloro-2-hydroxydiphenylether) as noted above.

Other antimicrobial agents suitable for combining with the present imidazolium polymer include (alternative names in brackets; numbers: Chemical Abstracts Registry):
(benzothiazol-2-ylthio)methyl thiocyanate (TCMTB) 21564-17-0
(benzyloxy)methanol 14548-60-8
(ethylenedioxy)dimethanol (Reaction products of ethylene glycol with paraformaldehyde (EGForm)) 3586-55-8
.alpha.,.alpha.',.alpha."-trimethyl-1,3,5-triazine-1,3,5(2H,4H,6H)-triethanol (HPT) 25254-50-6
1,2-benzisothiazol-3(2H)-one (BIT) 2634-33-5
1,3-bis(hydroxymethyl)-5,5-dimethylimidazolidine-2,4-dione (DMDMH) 6440-58-0
1-[2-(allyloxy)-2-(2,4-dichlorophenyl)ethyl]-1H-imidazole (Imazalil) 35554-44-0
1-[[2-(2,4-dichlorophenyl)-4-propyl-1,3-dioxolan-2-yl]methyl]-1H-1 ,2,4-triazole (Propiconazole) 60207-90-1
2,2-dibromo-2-cyanoacetamide (DBNPA) 10222-01-2
2,2',2"-(hexahydro-1,3,5-triazine-1,3,5-triyl)triethanol (HHT) 4719-04-4
2,2'-dithiobis[N-methylbenzamide] (DTBMA) 2527-58-4
2-bromo-2-(bromomethyl)pentanedinitrile (DBDCB) 35691-65-7
2-Butanone, peroxide 1338-23-4
2-butyl-benzo[d]isothiazol-3-one (BBIT) 4299-07-4
2-methyl-2H-isothiazol-3-one (MIT) 2682-20-4
2-octyl-2H-isothiazol-3-one (OIT) 26530-20-1
2-Phenoxyethanol 122-99-6
Phenoxypropanol 4169-04-4
2-thiazol-4-yl-1H-benzoimidazole (Thiabendazole) 148-79-8
3,3'-methylenebis[5-methyloxazolidine] (Oxazolidin/MBO) 66204-44-2
3-(4-isopropylphenyl)-1,1-dimethylurea/ Isoproturon 34123-59-6
3-iodo-2-propynylbutylcarbamate (IPBC) 55406-53-6
4,5-Dichloro-2-octylisothiazol-3(2H)-one (4,5-Dichloro-2-octyl-2H-isothiazol-3-one (DCOIT)) 64359-81-5
5-chloro-2-(4-chlorphenoxy)phenol (DCPP) 3380-30-1
6-(phthalimido)peroxyhexanoic acid (PAP) 128275-31-0
7a-ethyldihydro-1H,3H,5H-oxazolo[3,4-c]oxazole (EDHO) 7747-35-5
Acrolein 107-02-8
Active Chlorine: manufactured by the reaction of hypochlorous acid and sodium hypochlorite produced in situ
Alkyl (C12-16) dimethylbenzyl ammonium chloride (ADBAC/BKC (C12-16))68424-85-1
Alkyl (C12-18) dimethylbenzyl ammonium chloride (ADBAC (C12-18)) 68391-01-5
Alkyl (C12-C14) dimethylbenzylammonium chloride (ADBAC (C12-C14)) 85409-22-9
Alkyl (C12-C14) ethylbenzylammonium chloride (ADEBAC (C12-C14)) 85409-23-0
Allyl isothiocyanate 57-06-7
Amines, C10-16-alkyldimethyl, N-oxides 70592-80-2
Amines, N-C12-C14 (even-numbered)-alkyl- trimethylenedi-, reaction products with chloroacetic acid (Ampholyt 20) 139734-65-9
Ammonium bromide 12124-97-9
Ammonium sulphate 7783-20-2
Azoxystrobin 131860-33-8
Benzoic acid 65-85-0
Biphenyl-2-ol 90-43-7
Bis(1-hydroxy-1 H-pyridine-2-thionato- O,S)copper (Copper pyrithione)14915-37-8 Bromine chloride 13863-41-7
Bromoacetic acid 79-08-3
Bromochloro-5,5-dimethylimidazolidine-2,4-dione (BCDMH/ Bromochlorodimethylhydantoin) 32718-18-6
Bronopol 52-51-7
Calcium hydroxide (Ca dihydroxide/caustic lime/hydrated lime/slaked lime) 1305-62-0 Calcium hypochlorite 7778-54-3
Calcium magnesium oxide/dolomitic lime 37247-91-9
Calcium magnesium tetrahydroxide (Ca Mg hydroxide/hydrated dolomitic lime) 39445-23-3
Calcium oxide (lime/burnt lime/quicklime) 1305-78-8
Carbendazim 10605-21-7
Cetylpyridinium chloride 123-03-5
Chloramin B 127-52-6
Chlorine 7782-50-5
Chlorine dioxide 10049-04-4
Chlorocresol 59-50-7
Cinnamaldehyde/3-phenyl-propen-2-al (Cinnamic aldehyde) 104-55-2 cis-1-(3-chloroallyl)-3,5,7-triaza-1- azoniaadamantane chloride (cis CTAC) 51229-78-8
Citric acid 77-92-9
Clorophene (Chlorophene) 120-32-1
Copper 7440-50-8
Copper sulphate pentahydrate 7758-99-8
Copper thiocyanate 1111-67-7
Cyanamide 420-04-2
D-gluconic acid, compound with N,N"-bis(4-chlorophenyl)-3,12-diimino-2,4,11,13-tetraazatetradecanediamidine(2:1) (CHDG) 18472-51-0
Decanoic acid 334-48-5
Dichloro-N-[(dimethylamino)sulphonyl] fluoro-N-(ptolyl)methanesulphenamide (Tolylf luanid) 731-27-1
Dicopper oxide 1317-39-1
Didecyldimethylammonium chloride (DDAC (C8-10)) 68424-95-3
Didecyldimethylammonium chloride (DDAC) 7173-51-5
Dimethyloctadecyl[3-(trimethoxysilyl)propyl]ammonium chloride 27668-52-6
Dimethyltetradecyl[3-(trimethoxysilyl)propyl]ammonium chloride 41591-87-1
Disodium peroxodisulphate/Sodium persulphate 7775-27-1
Diuron 330-54-1
Dodecylguanidine monohydrochloride 13590-97-1
Ethanol 64-17-5
Ethylene oxide 75-21-8
Fludioxonil 131341-86-1
Formaldehyde 50-00-0
Formic acid 64-18-6
Glutaral (Glutaraldehyde) 111-30-8
Glycolic acid 79-14-1
Glyoxal 107-22-2
Hexa-2,4-dienoic acid (Sorbic acid) 110-44-1
Hydrochloric acid
Hydrogen peroxide 7722-84-1
Iodine 7553-56-2
L-(+)-lactic acid 79-33-4
Magnesium monoperoxyphthalate hexahydrate (MMPP) 84665-66-7
MBIT 2527-66-4
Mecetronium ethyl sulphate (MES)3006-10-8
Medetomidine 86347-14-0
Metam-sodium 137-42-8
Methenamine 3-chloroallylochloride (CTAC) 4080-31-3
Methylene dithiocyanate 6317-18-6
Mixture of 5-chloro-2-methyl-2H- isothiazol-3-one (EINECS 247-500-7) and 2-methyl-2H-isothiazol-3-one (EINECS 220-239-6) (Mixture of CMIT/MIT) 55965-84-9
Monohydro chloride of polymer of N,N"'-1,6-hexanediylbis[N'-cyanoguani-dine]
(EINECS 240-032-4) and hexamethylenediamine (EINECS 204-679-6)/ Polyhexamethylene biguanide (monomer:1,5-bis(trimethylen)-guanylguanidinium monohydrochloride) (PHMB) 27083-27-8
Monolinuron 1746-81-2
N,N'-methylenebismorpholine (MBM) 5625-90-1
N-(3-aminopropyl)-N-dodecylpropane-1,3-diamine (Diamine) 2372-82-9
N-(Dichlorofluoromethylthio)-N',N'-dimethyl-N-phenylsulfamide (Dichlofluanid) 1085-98-9
N-(trichloromethylthio)phthalimide (Folpet) 133-07-3
Nonanoic acid (Pelargonic acid) 112-05-0
N'-tert-butyl-N-cyclopropyl-6-(methylthio)-1,3,5-triazine-2,4-diamine (Cybutryne) 28159-98-0
Octanoic acid 124-07-2
Ozone 10028-15-6
p-[(diiodomethyl)sulphonyl]toluene 20018-09-1
Pentapotassium bis(peroxymonosulphate) bis(sulphate) 70693-62-8
Peracetic acid 79-21-0
Peroxyoctanoic acid 33734-57-5
Poly(oxy-1,2-ethanediyl),alpha-[2-(dide- cylmethylammonio)ethyl]-omega-hydroxy-, propanoate (salt) (Bardap 26) 94667-33-1
Polymer of N-Methylmethanamine (EINECS 204-697-4 with (chloromethyl) oxirane (EINECS 203-439-8)/Polymeric quaternary ammonium chloride (PQ Polymer) 25988-97-0
Polyvinylpyrrolidone iodine 25655-41-8
Potassium (E,E)-hexa-2,4-dienoate (Potassium Sorbate) 24634-61-5
Potassium 2-biphenylate 13707-65-8
Potassium dimethyldithiocarbamate 128-03-0
Propan-1-ol 71-23-8
Propan-2-ol 67-63-0
Pyridine-2-thiol 1-oxide, sodium salt (Sodium pyrithione) 3811-73-2
Pyrithione zinc (Zinc pyrithione) 13463-41-7
Quaternary ammonium compounds, benzyl-C12-18-alkyldimethyl, salts with 1,2-benzisothiazol-3(2H)-one 1,1-dioxide 68989-01-5
Reaction mass of titanium dioxide and silver chloride
Reaction products of 5,5-dimethylhydantoin, 5-ethyl-5-methylhydantoin with bromine and chlorine (DCDMH)
Reaction products of: glutamic acid and N-(C12-C14-alkyl)propylenediamine (Gluco-protamin) 164907-72-6
Salicylic acid 69-72-7
Silver 7440-22-4
Silver adsorbed on silicon dioxide
Silver copper zeolite 130328-19-7
Silver nitrate 7761-88-8
Silver phosphate glass 308069-39-8
Silver sodium hydrogen zirconium phosphate 265647-11-8
Silver zeolite
Silver zinc zeolite 130328-20-0
Sodium 2-biphenylate 132-27-4
Sodium bromide 7647-15-6
Sodium dichloroisocyanurate dihydrate 51580-86-0
Sodium dimethyldithiocarbamate 128-04-1
Sodium hypochlorite 7681-52-9
Sodium metabisulfite 7681-57-4
Sodium N-(hydroxymethyl)glycinate 70161-44-3
Sodium p-chloro-m-cresolate 15733-22-9
Sulphur dioxide 7446-09-5
Symclosene 87-90-1
tebuconazole 107534-96-3
Terbutryn 886-50-0
Tetrachlorodecaoxide complex (TCDO) 92047-76-2
Tetrahydro-1,3,4,6-tetrakis(hydroxymethyl)imidazo[4,5-d]imidazole-2,5(1H,3H)-dione (TMAD) 5395-50-6
Tetrahydro-3,5-dimethyl-1,3,5-thiadia-zine-2-thione (Dazomet) 533-74-4 Tetrakis(hydroxymethyl)phosphonium sulphate (2:1) (THPS) 55566-30-8
Thiram 137-26-8
Tosylchloramide sodium (Tosylchloramide sodium - Chloramin T) 127-65-1 Tralopyril 122454-29-9
Triclosan 3380-34-5
Troclosene sodium 2893-78-9
Zineb12122-67-7;
also of importance are Pyrithiones, Dimethyldimethylol Hydantoin,Methylchloroisothiazolinone/methylisothiazolinone, Sodium Sulfite, Sodium Bisulfite, Imidazolidinyl Urea, Diazolidinyl Urea, Benzyl Alcohol, 2-Bromo-2-nitropropane-1,3-diol, formaldehyde, lodopropenyl Butylcarbamate, Chloroacetamide, Methanamine, Methyldibromonitrile Glutaronitrile (1,2-Dibromo-2,4-dicyanobutane), Glutaraldehyde, 5-bromo-5-nitro-1,3-dioxane, Phenethyl Alcohol, o-Phenylphenol/s, for example, commonly encountered compounds such as farnesol, parfumes, phenoxyethanol, quaternary compounds, triclocarban, organic acids such as benzoic acid or sorbic acid, biguanides such as poly-(hexamethylene biguanide) hydrochloride phenoxypropanol, benzalkonium chloride, cetrimonium bromide or benzethonium chloride or salicylic acid and the like. Another class of antibacterial agents, which can additionally be used, are the so-called "natural" antibacterial actives, referred to as natural essential oils. Additional active agents are antibacterial metal salts. This class generally includes salts of metals in groups 3b-7b, 8 and 3a-5a. Specifically are the salts of aluminum, zirconium, zinc, silver, gold, copper, lanthanum, tin, mercury, bismuth, selenium, strontium, scandium, yttrium, cerium, praseodymiun, neodymium, promethum, samarium, europium, gadolinium, terbium, dysprosium, holmium, erbium, thulium, ytterbium, lutetium and mixtures thereof.

For example, the composition of the invention may comprise such further auxiliaries typically in amounts from the range 0.01 - 2 wt%, such as 0.05 - 2 wt% further antimicrobial agents, 0.1 -1 wt% anti-foaming agents, and 0.1 -1 wt% colorants, or in higher amounts where appropriate, such as 5-15 % by weight of anti-freezing agents.

The invention also pertains to a method for combating harmful organisms or for protecting human beings, animals, materials or processes from the effects of these harmful organisms, wherein the habitat of the harmful organism or the human being, animal or material to be protected is brought into contact with a biocide composition or the biocide composition is employed in said process, wherein the biocide composition comprises at least one polymeric, ionic compound of defined molecular weight and comprising imidazolium groups as defined above, provided that the method is not a method for treatment of the human or animal body by surgery or therapy or diagnostic method practised on the human or animal body. "Harmful organism" means any organism, including pathogenic agents, which have an unwanted presence or a detrimental effect on humans, their activities or the products they use or produce, or on animals, plants or the environment. The present method is preferably used for combating harmful organisms selected from bacteria, fungi, algae, viruses and mycoplasma, especially bacteria and fungi.

The invention thus includes a method for disinfecting non-living (i.e. inanimate) objects and surfaces to destroy microorganisms that are living on said surfaces by treating said objects or surfaces with a composition as described above.

The invention further includes a method for preserving inanimate materials, such as plastics, foams, liquids such as household or cosmetic formulations, and preventing growth of microorganisms within such materials, by adding, especially admixing, an effective amount of the polymeric ionic compound of defined molecular weight and comprising imidazolium groups as defined above to said materials. Effective amounts for such applications are typically 0.0001 to about 5%, especially 0.001 to about 2% b.w. of the material or formulation to be preserved.

According to the classification of the EU Biocidal Products Directive, biocides are classified in 23 product types (i.e. application categories), which may roughly be categorised in the following main groups: general biocidal products, disinfectants, preservatives, pest control, anti-fouling products. The present invention includes a product selected from general biocidal products, disinfectants, preservatives, pest control, anti-fouling products, each of which is characterized by containing the present polyimidazolium compound as an antimicrobial agent.

The invention also pertains to a process for achieving an antimicrobial effect, especially an antibacterial and/or antifungal effect, on a hard surface, by contacting said surface with a liquid formulation as described above, which formulation contains the present polyimidazolium compound.

The invention includes the use of the polymeric, ionic compound defined above as a biocide in compositions for various applications, examples of which are
- a plant protection composition; preferably a fungicidal composition, or
- a personal care composition, or
- a home care composition, or
- a composition used for industrial or institutional or hospital disinfection, or
- a material protection composition, or
- an in-can preservation composition, or
- a composition used for microbial control of industrial processes such as pulp and paper making, water treatment, oil and gas exploration, or
- a pharmaceutical composition.

Further examples are hair-treatment compositions, skin-cleansing compositions, compositions for the care and protection of the skin, nail care compositions and preparations for decorative cosmetics.

### Personal Care

The invention accordingly relates also to a personal care preparation comprising at least one imidazolium polymer as described above, e.g. of formula I, and cosmetically tolerable carriers or adjuvants.

The personal care composition typically comprises
A) at least one polymeric, ionic compound comprising imidazolium groups (imidazolium compound) as defined above,
B) optionally at least one cosmetically acceptable active ingredient, and
C) at least one cosmetically acceptable auxiliary.

The composition may, for example, be formulated in the form of
- an emulsion, preferably selected from as a W/O, O/W, O/W/O, W/O/W or PIT emulsion and microemulsions,
- a suspension,
- a gel,
- an oil, a cream, milk or lotion,
- a powder, a lacquer, a tablet or make-up,
- a stick,
- a spray or an aerosol,
- a foam,
- a paste, mousse or an ointment
- an aqueous or alcoholic solution or liquid.

The polymers of the invention are accordingly suitable as antimicrobial active substances and preservatives in personal care preparations, including cosmetic products and household products, for example shampoos, bath additives, skin and hair care preparations, liquid and solid soaps, lotions, creams, deodorants and other aqueous or alcoholic solutions. The invention accordingly relates also to a personal care preparation comprising a non-ionic surfactant.

Depending upon the form of the personal care preparation, the amount of imidazolium polymer of the present invention and optionally surfactant will vary and may comprise a majority of the preparation. Typically, the personal care preparation according to the invention contains from about 0.01 to about 30% by weight, for example from about 0.1 to about 20% by weight, for example from about 0.1 to about 15% by weight based on the total weight of the composition, of a mixture of the ionic imidazolium polymer and a surfactant, besides and cosmetically tolerable adjuvants.

The antimicrobial agents of the present invention can be used as ingredients in a wide variety of cosmetic or personal care preparations. Suitable personal care compositions include skin-care preparations, e.g. skin-washing and cleansing preparations in the form of tablets, liquid soaps, bar soaps, syndets, washing gels, soapless detergents or washing pastes, bath preparations, e.g. liquid (foam baths, milks, oils, shower preparations) or solid bath preparations, e.g. bath cubes and bath salts; skin-care preparations, e.g. skin emulsions, multi-emulsions, powders, sprays or skin oils; cosmetic personal care preparations, e.g. facial make-up in the form of day creams or powder creams, face powder (loose or pressed), rouge or cream make-up, eye-care preparations, e.g. eyeshadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers; foot-care preparations, e.g. foot baths, foot powders, foot creams or foot balsams, special deodorants and antiperspirants or callus-removing preparations; light-protective preparations, such as sun milks, lotions, creams or oils, sunblocks or tropicals, pre-tanning preparations or after-sun preparations; skin-tanning preparations, e.g. self-tanning creams; depigmenting preparations, e.g. preparations for bleaching the skin or skin- lightening preparations; insect-repellents, e.g. insect-repellent oils, lotions, sprays or sticks; deodorants, such as deodorant sprays, deodorant aerosols, pump-action sprays, deodorant gels, sticks or roll-ons, also water-free deodorant aerosols or sticks; antiperspirants, e.g. antiperspirant sticks, creams or roll-ons, also water-free antiperspirant aerosols and water-free antiperspirant sticks; preparations for cleansing and caring for blemished skin, e.g. synthetic detergents (solid or liquid), peeling or scrub preparations or peeling masks; hair-removal preparations in chemical form (depilation), e.g. hair-removing powders, liquid hair-removing preparations, cream- or paste-form hair-removing preparations, hair-removing preparations in gel form or aerosol foams; shaving preparations, e.g. shaving soap, foaming shaving creams, non-foaming shaving creams, foams and gels, pre-shave preparations for dry shaving, aftershaves or aftershave lotions; fragrance preparations, e.g. fragrances (eau de Cologne, eau de toilette, eau de parfum, parfum de toilette, perfume), perfume oils or perfume creams; cosmetic hair-treatment preparations, e.g. hair-washing preparations in the form of shampoos and conditioners, hair-care preparations, e.g. pretreatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hairsetting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidising dyes, or natural hair colorants, such as henna or camomile, antidandruff preparations in the form of shampoos, conditioners, hair tonics, styling creams or gels or treatments packs; oral care preparations for the treatment of oral mucous membranes, gums and teeth such as pastes, gels, lotions, powders, mouth washes and sprays. The composition of the invention may also be included in products for intimate hygiene uses including feminine hygiene products such as lotions, gels, creams, washes, powders, sprays.

Especially preferred are the preparations like skin-care preparations, bath preparations, cosmetic personal care preparations, foot-care preparations; light-protective preparations, skin-tanning preparations, depigmenting preparations, insect-repellents, deodorants, antiperspirants, preparations for cleansing and caring for blemished skin, hair-removal preparations in chemical form (depilation), shaving preparations, fragrance preparations or cosmetic hair-treatment preparations.

In order to achieve the mildness required of the antimicrobial composition of the present invention, optional ingredients to enhance the mildness to the skin can be added. These ingredients are well known in the filed and include synthetic and naturally occurring polymers, co-surfactants and moisturizers.

For example, lipophilic skin conditioning agents may be present, for example, hydrocarbon oils and waxes, silicones, fatty acid derivatives, cholesterol, cholesterol derivatives, di- and tri-glycerides, vegetable oils, vegetable oil derivatives, liquid nondigestible oils such as those described in US Pat 3,600,186; 4,005,195 and 4,005,196, all of which are herein incorporated by reference, or blends of liquid digestible or nondigestible oils with solid polyol polyesters such as those described in US Pat 4,797,300; 5,306,514; 5,306,516 and 5,306,515, all of which are herein incorporated by reference, and acetoglyceride esters, alkyl esters, alkenyl esters, lanolin and its derivatives, milk tri-glycerides, wax esters, beeswax derivatives, sterols, phos-pholipids and mixtures thereof.

Nonlimiting examples of silicone useful herein are described in US Pat 5,011,681, to Ciotti et al., issued Apr. 30, 1991, which is incorporated by reference.

Nonlimiting examples of naturally occuring oils include castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil and sesame oil, vegetable oils and vegetable oil derivatives; coconut oil and derivatized coconut oil, cottonseed oil and derivatized cottonseed oil, jojoba oil, cocoa butter, and the like.

Acetoglyceride esters may be used and an example is acetylated monoglycerides.

When a lipophilic skin moisturizing agent is employed as the mildness enhancer in the antimicrobial compositions herein, a stabilizer may also be included. The stabilizer is used to form a crystalline stabilizing network in the liquid composition that prevents the lipophilic skin moisturizer agent droplets from coalescing and phase splitting in the product. The network exhibits time dependent recovery of viscosity after shearing (e.g., thixotropy).

The stabilizers used herein are not surfactants. The stabilizers provide improved shelf and stress stability. Typical hydroxyl-containing stabilizers include 12-hydroxystearic acid, 9,10-dihydroxystearic acid, tri-9,10-dihydroxystearin and tri-12-hydroxystearin (hydrogenated castor oil is mostly tri-12-hydroxystearin).

Alternatively, the stabilizer employed in the antimicrobial compositions herein can comprise for example a polymeric thickener, a C₁₀-C₂₂ ethylene glycol fatty acid ester, dispersed amorphous silica, dispersed smectite clay such as bentonite and hectorite, a fatty acid or fatty alcohol.

The antimicrobial compositions of the present invention can comprise a wide range of optional ingredients. The CTFA International Cosmetic Ingredient Dictionary, Sixth Edition, 1995, which is incorporated by reference herein in its entirety, describes a wide variety of materials commonly used in the cosmetic and personal care industry suitable for use in the compositions of the present invention. Nonlimiting examples of functional classes of ingredients are described at page 537 of this reference.

Examples of these functional classes include: abrasives, anti-acne agents, anticaking agents, antioxidants, binders, biological additives, bulking agents, chelating agents, chemical additives, colorants, cosmetic astringents, cosmetic biocides, denaturants, drug astringents, emulsifiers, external analgesics, film formers, fragrance components, humectants, opacifying agents, plasticizers, preservatives, propellants, reducing agents, skin bleaching agents, skin-conditioning agents (emollient, humectants, miscellaneous, and occlusive), skin protectants, solvents, foam boosters, hydrotropes, solubilizing agents, suspending agents (nonsurfactant), sunscreen agents, ultraviolet light absorbers, and viscosity increasing agents (aqueous and nonaqueous). Examples of other functional classes of materials useful herein that are well known to one of ordinary skill in the art include solubilizing agents, sequestrants, and keratolytics, and the like.

The personal care preparation according to the invention may exist in a wide variety of presentation forms, for example a water-in-oil or oil-in-water emulsion, an alcoholic or alcohol-containing formulation, a vesicular dispersion of an ionic or non-ionic amphiphilic lipid, a gel, a solid stick, cream, milk or lotion, a powder, a lacquer, a tablet or make-up, a stick, a spray or an aerosol, a foam, or a paste and all kinds of microemulsions.

As a water-in-oil (W/O) or oil-in-water emulsion (O/W), the cosmetically tolerable adjuvant typically contains from 5 to 50% of an oil phase, from 5 to 20% of an emulsifier and from 30 to 90% water. The oil phase may comprise any oil suitable for cosmetic formulations, for example one or more hydrocarbon oils, a wax, a natural oil, a silicone oil, a fatty acid ester or a small chain or fatty alcohol including mono- and poly-ols, e.g., ethanol, isopropanol, propylene glycol, hexylene glycol, glycerol and sorbitol.

The aqueous phase contains for example ingredients such as alcohols, diols or polyols or their ethers as well as one or more thickeners for example of the groups of silicium dioxide, aluminium silicates, polysaccharides or derivatives thereof for example hyaluronic acid, xanthan gum, hydroxypropylmethylcellulose, polyacrylates.

The compositions of the invention may also include various thickeners, such as crosslinked acrylates, nonionic polyacrylamides, xanthan gum, guar gum, gellan gum, and the like; polyalkyl siloxanes, polyaryl siloxanes, and aminosilicones.

The specific examples of the suitable thickening silicon compounds include polydimethylsiloxane, phenylsilicone, polydiethylsiloxane, and polymethylphenylsiloxane. Some of the suitable silicon compounds are described in European Patent Application EP 95,238 and US Pat 4,185,017, which patent is incorporated herein by reference. The compositions of the invention may also include silicone polymer materials, which provide both style retention and conditioning benefits to the hair. Such materials are described in US Pat 4,902,499, which is incorporated herein by reference.

Further cosmetically tolerable adjuvants include emulsifiers, perfume oils, rheology modifiers (thickeners), hair polymers, hair and skin conditioners, water-soluble or dispersible silicone-comprising polymers, bleachers, gelling agents, care agents, colorants, tinting agents, tanning agents, dyes, pigments, antidandruff agents, sunscreen agents, deodorizing active substances, vitamins, plant extracts, bodying agents, humectants, refatting agents, collagen, protein hydrolysates, lipids, antioxidants, anti-foaming agents, antistatic agents, emollients, softeners, etc.

Further suitable cosmetically active substances for use within the composition of the invention are, for example, skin and hair pigmentation agents, tanning agents, bleaches, keratin-hardening substances, antimicrobial active substances, photofilter active substances, repellent active substances, hyperemic substances, keratolytic and keratoplastic substances, antidandruff active substances, antiphlogistics, keratinizing substances, active substances which act as antioxidants and/or as free-radical scavengers, skin moisturizing or humectant substances, refatting active substances, deodorizing active substances, sebostatic active substances, plant extracts, antierythimatous or antiallergic active substances and mixtures thereof; artificially skin-tanning active substances which are suitable for tanning the skin without natural or artificial irradiation with UV rays are, for example, dihydroxyacetone, alloxan and walnut shell extract; suitable keratin-hardening substances are generally active substances as are also used in antiperspirants, such as, for example, potassium aluminum sulfate, aluminum hydroxychloride, aluminum lactate, etc.; further antimicrobial active substances are as defined further above, these include, for example, customary preservatives known to the person skilled in the art, such as p-hydroxybenzoates, imidazolidinylurea, formaldehyde, sorbic acid, benzoic acid, salicylic acid, etc.; deodorizing substances are, for example, zinc ricinoleate, triclosan, undecylenic acid alkylolamides, triethyl citrate, chlorhexidine etc.; suitable photofilter active substances are substances which absorb UV rays in the UV-B and/or UV-A region; suitable UV filters include hydroxyphenyl triazines, p-aminobenzoic esters, cinnamic esters, benzophenones, camphor derivatives, and pigments which stop UV rays, such as titanium dioxide, talc and zinc oxide; suitable repellent active substances are compounds which are able to keep or drive certain animals, in particular insects, away from people, these include, for example, 2-ethyl- 1 ,3-hexanediol, N,N-diethyl-m-toluamide, etc.; suitable hyperemic substances which stimulate blood flow in the skin are, for example, essential oils, such as dwarf-pine, lavender, rosemary, juniper berry, horse chestnut extract, birch leaf extract, hay flower extract, ethyl acetate, camphor, menthol, peppermint oil, rosemary extract, eucalyptus oil, etc. Suitable keratolytic and keratoplastic substances are, for example, salicylic acid, calcium thioglycolate, thioglycolic acid and its salts, sulfur, etc.; suitable antidandruff active substances are, for example, sulfur, sulfur polyethylene glycol sorbitan monooleate, sulfur ricinol polyethoxylate, zinc pyrithione, aluminum pyrithione, etc. Suitable antiphlogistics, which counteract skin irritations, are, for example, allantoin, bisabolol, dragosantol, camomile extract, panthenol, etc.

The compositions according to the invention can comprise at least one further polymer. These include, very generally, anionic, cationic, amphoteric and neutral polymers. Suitable anionic polymers for the personal care compositions according to the invention are generally all anionic polymers known for this application. The composition according to the invention preferably comprises at least one soluble or dispersed anionic polymer. The anionic polymers that may be employed include, but are not limited to, polymers comprising groups derived from carboxylic acids, sulfonic acids or phosphoric acids. Preferably, the anionic polymers have a number-average molecular mass in a range from 500 to 5 000 000.
Suitable additional cationic polymers are polymers different from the imidazolium compounds according to the invention; they may be chosen in principle from all cationic polymers known to a person skilled in the art as suitable for cosmetic compositions. Cationic polymers for compositions for improving the cosmetic properties of the hair are for example those described in patent applications EP-A-0 337 354 FR-A-2 270 846, FR-2 383 660, FR-2 598 61 1 , FR-2 470 596 and FR-2 519 863. For the purposes of the present invention, the term "cationic polymer" denotes any polymer comprising at least one cationic group or at least one cationogenic group that may be ionized into a cationic group. The at least one cationic polymer may be chosen from those containing units comprising primary, secondary, tertiary, and/or quaternary amine groups that either may form part of the main polymer chain or may be borne by a side substituent directly attached thereto.

The personal care composition according to the invention can also be a composition that contains the present imidazolium compound as preservative.

In addition to the abovementioned constituents, the personal care compositions according to the invention may also comprise at least one surface-active substance, including dispersing agents and wetting agents, besides surfactants already described above.

### Home Care and Industrial & Institutional Applications

The invention includes a home care preparation for private consumers or a preparation for professional users e.g. in Industrial & Institutional (I&I) areas (e.g. hospitals, old people homes, kindergardens, restaurants, can-teens, kitchens, production areas and production equipment for cosmetic and pharma-ceutical products, production areas and production equipment for food and beverages, animal stables etc.) comprising at least one imidazolium polymer as described above, e.g. of formula I, and suitable carriers and/or adjuvants.

The composition optionally contains at least one further microbicidal compound different from the present polyimidazolium compound and/or optionally at least one further active ingredient and/or auxiliary. The home care or I&I composition according to the invention can be a composition that is effective against various microorganisms. According to this variant, the imidazolium compound itself may act as active ingredient. Accordingly, in such a composition the use of a further active ingredients and/or auxiliaries is only optional. The home care or I&I composition according to the invention can also be a composition that contains the present imidazolium compound as preservative.

A typical home care or I&I composition according to the invention contains the present imidazolium compound, optionally at least one further microbicidal compound, and at least one further component selected from non-ionic surfactants, anionic surfactants, amphoteric surfactants, water, alcohols and a combination thereof. The home care compositions can include additional components such as enzymes, bleaches, whiteners, color care agents, fabric softeners, suds suppressors, dispersants, dye transfer inhibitors, chelating agents, aerosol propellants, gelling agents, thickening agents and a combination thereof.

The home care or I&I composition according to the invention can be formulated in a variety of ways and may include a hydrophilic phase, a hydrophobic phase and optionally at least one emulsifying agent. The home care or I&I composition may be in the form of a liquids, semi-solid, paste, gel, bar, tablet, spray, foam, powder or granules. For the purpose of the invention the term "home care composition" means a composition for use in the general environment of human beings and is further described in the following. Home care compositions are generally nontoxic when applied in the vicinity of human beings, for example to fabrics and other items used by humans, when applied to surfaces used by, or in the vicinity of, humans, or when applied to spaces occupied by humans.

A further aspect of the invention is a method of using a home care or I&I composition, as defined above and in the following, by applying the composition to an article, surface or space. Exemplary articles, surfaces and spaces include clothes, furniture fabrics, rugs and carpets, draperies, dishes and cooking utensils, grills, ovens, and other items used by humans. The term "surface" includes hard surfaces in the human environment, such as floors, glass surfaces (such as glass windows, doors and countertops), other counter surfaces, bath, toilet bowl, sink and other bathroom surfaces. The term "space" includes the interior portion of buildings occupied by humans, including the air contained therein.

Advantageously, a home care or I&I composition comprising at least one imidazolium compound of the invention possesses effective antimicrobial preservative properties. Further, a home care or I&I composition comprising the imidazolium compound also confers an antimicrobial effect on articles, surfaces or spaces to which it is applied. Home care and I&I compositions according to the invention include:
- surface cleaning compositions (for example, glass, floor, counter, bath, toilet bowl, sink, appliance and furniture cleaning compositions);
- deodorants (for example, solid, liquid and spray deodorants air and/or surface deodorants);
- disinfectants (for example, spray and solid air disinfectants (including gel); and spray, solid, liquid and paste surface disinfectants);
- waxes and other surface protecting and/or polishing compositions;
- laundry compositions (such as powder and liquid detergents, fabric softeners, other after-rinses, whiteners) for domestic, commercial, institutional and industrial laundering including special uses such as "On Premises Laundry" (OPL);
- rug shampoos, carpet sprays.

Typical products comprising the present imidazolium polymer in the Home Care and I&I area include for example hard surface cleaners, floor cleaners, hard surface or floor disinfectants, all purpose cleaner, dish washing agents, bathroom cleaners or hand disinfectants. The compositions and products of the invention may advantageously be used also for cleaning and disinfection uses in industrial or institutional (I&I) settings, which settings explicitly include hospital disinfection, cleaning and sanitation including Health Care, Building Care, Food service & kitchen hygiene, Food and beverage processing or Laundry Care (commercial, institutional and industrial laundering), and include product types such as hard surface cleaners and disinfectants, floor cleaners and disinfectants, hand disinfectants, hand sanitizers and hand soaps, cleaning-in-place (CIP)-cleaning products, laundry detergents, fabric softeners and after rinses.

Preferably, the home care and I&I composition comprises the present imidazolium polymer and, if present, further adjuvants or active components in a fraction of from about 0.001 to 50% by weight, particularly preferably 0.01 to 30% by weight, in particular 0.1 to 20% by weight, based on the total weight of the composition. Such formulations may be ready-for-use compositions or concentrates, and typically comprise further additional components as described above. Concentrated formulations of the present imidazolium polymer contain it preferably in amounts ranging from 10 to about 70 percent, by total weight of the concentrate.

### Pharmaceutical composition

A further aspect of the invention is a pharmaceutical composition comprising
A) at least one imidazolium compound as defined above,
B) optionally at least one further microbicidal compound different from the compounds of component (A),
C) optionally at least one pharmaceutically acceptable active ingredient, and
D) optionally at least one pharmaceutically acceptable excipient.

The pharmaceutical composition typically comprises the present imidazolium compound as defined above (A), at least one pharmaceutically acceptable carrier or excipient (D), and, optionally, at least one pharmaceutically active ingredient (C) and/or further microbicidal compound different from the present imidazolium compounds (B).

Preferably, the pharmaceutical composition comprises the components A) and, if present, B) in a fraction of from about 0.001 to 50% by weight, particularly preferably 0.01 to 30% by weight, in particular 0.1 to 20% by weight, based on the total weight of the composition.

The pharmaceutical composition of the invention is preferably used for application on the skin, mucous membranes (oral care and intimate uses) and hair.

The pharmaceutical composition may, for example, be formulated in the form of
- an emulsion, preferably selected from as a W/O, O/W, O/W/O, W/O/W or PIT emulsion and microemulsions,
- a suspension,
- a gel,
- an oil, a cream, milk or lotion,
- a powder, a lacquer, a tablet or make-up,
- a stick,
- a spray or an aerosol,
- a foam,
- a paste, mousse or an ointment,
- an aqueous or alcoholic solution or liquid.

The pharmaceutical composition is suitable for administering in principle any type of active pharmaceutical ingredient C). These include vitamins, cytostatics, in particular taxol, antibiotics, antimycotics, fungicides, chemother-apeutics, sulfonamides, enzyme preparations and transport proteins, enzyme inhibitors, emetics, diagnostics, corticoids, antiinflammatories, antiallergics, anthelmintics, Exam-ples of suitable active ingredients C) are: virustatics, acyclovir, 5-aminosalicylic acid, cefalosporins, clarithromycin, clavulanic acid, clindamycin, cotrimoxazole, vitamin D and derivatives of vitamin D, cysteine, ciclosporin, dimethicone, diclofenac, glycoside antibiot-ics, econazole, peptide antibiotics, macrolide antibioticsfenticonazole, gyrase inhibitor, fluconazole, fusidic acid, galantamine, ganciclovir, gentamicin, ginkgo, glucagon, glu-cosamine and glucosamine derivatives, glutathione, glycerol and glycerol derivatives, hyaluronic acid, salicylates, iodine and iodine derivatives, isoconazole, itraconazole, ke-toconazole, lipoic acid and lipoic acid derivatives, metamizole, miconazole, mitomycin, evening primrose, natamycin, nystatin, oral penicillins, plant extracts, rifampicin, silicates, sulfonamides propionic acid derivatives and the like.

The active ingredients can, if desired, also be used in the form of their pharmaceutically acceptable salts or derivatives, and in the case of chiral active ingredients it is possible to employ both optically active isomers and racemates or mixtures of diastereoisomers. The compositions of the invention can, if desired, also comprise two or more active pharmaceutical ingredients.

The formulation base of pharmaceutical compositions of the invention preferably comprises pharmaceutically acceptable excipients D). Pharmaceutically acceptable excipients are those known to be usable in the area of pharmacy, food technology and adjacent sectors, in particular the excipients listed in relevant pharmacopeias (e.g. DAB, Ph. Eur., BP, USP, JP) and others, whose properties do not stand in the way of physiological use. Suitable excipients D) may be: lubricants, wetting agents, emulsifying and suspending agents, antioxidants, anti-irritants, chelating agents, emulsion stabilizers, film formers, gel formers, odor-masking agents, resins, hydrocolloids, solvents, solubilizers, neutralizers, permeation promoters, pigments, colorants, stabilizers, disintegrants, dessicants, opacifiers, thickeners, waxes, plasticizers, flavors, sweeteners, excipients to reduce permeation etc. An arrangement concerning this is based on specialist knowledge as described for example in Fiedler, H. P. Lexikon der Hilfsstoffe fur Pharmazie, Kosmetik und angrenzende Gebiete, 4th edition, Aulendorf: ECV-Editio-Cantor-Verlag, 1996.

### Application of the present composition

Antimicrobial formulations of the invention are also applied in clean-in-place (CIP) applications. CIP applications are used in practice in the food and beverage industry, like in breweries, in the dairy industry, in the soft-drink and juice manufacturing industry, but also in the cosmetic and pharmaceutical industry.

As noted, the invention includes a product selected from the group consisting of home care formulation, a disinfectant of hard and/or soft surfaces, sanitary detergent of hard and/or soft surfaces, and product for clean in place (CIP), wherein said article comprises, preferably comprises at least 70 wt.-% of, still more preferably comprises at least 90 wt.-% of, yet more preferably consists of, the formulation as described herein. Accordingly the present invention is especially directed to a product selected from the group consisting of disinfectant, all purpose cleaner, dishwashing liquid, descaling agent, bath room cleaner, toilet bowl cleaner, floor cleaner, glass cleaner, kitchen cleaner, sanitary cleaner, furniture cleaner and product for clean in place (CIP), wherein said product comprises, preferably comprises at least 70 wt.-% of, still more preferably comprises at least 90 wt.-% of, yet more preferably consists of, the formulation as described herein.

Like the formulation also the products containing the formulation of the present invention can be used as concentrate that has to be diluted with water prior to use or are ready-to-use products, which are used as such, e.g. without dilution.

The terms used in the previous paragraphs are understood according to the knowledge of the skilled artisan in the respective technical field. For instance, a disinfectant is a substance that is applied to non-living (i.e. inanimate) objects to destroy microorganisms that are living on said objects. On the other hand, the clean in place is a method of cleaning and/or disinfecting the interior surfaces of pipes, vessels, process equipment, filters and associated fittings without disassembly. The clean in place product according to this invention is especially used for removing soils and disinfection in facilities for processing typically liquid product streams such as beverages, milk, juices, etc. The formulation of the invention can be used for disinfection of the interior surfaces in CIP after a separate cleaning step. The cleaning step is performed with a cleaning formulation that is not biocidal or disinfecting. It may however also be the case that the formulation of the invention is used for CIP-cleaning and CIP disinfection in one single step.

Depending on the end application the formulation may contain further ingredients making it ready for the desired end-use. Such ingredients are known to the skilled artisan and do not contribute to the present invention. Depending upon the form of the formulation used at the end it comprises, in addition to the components mentioned above further constituents, for example surfactants (surface active agents), pH regulators, buffering agents,hydrotropes, polymers, metal sequestering or metal chelating agents, colourings, perfume oils, thickening or solidifying agents (consistency regulators), emulsifiers, emollients, UV-absorbers, and antioxidants.

Within the context of the invention, cleaning compositions or cleaners for inanimate materials are:
- compositions for cleaning and/or disinfecting hard surfaces, such as all-purpose cleaners, floor cleaners, dishwashing detergents for manual or automatic dishwashing, glass cleaners, kitchen cleaners, bath and sanitary cleaners, WC cleaners, disinfectant cleaners,
- cleaning and care compositions for textiles and laundry such as detergents, fabric softeners, stain removers.

Within the context of the invention, cleaning compositions or cleaners for use in an antimicrobial treatment of the human or animal body, especially of the skin, oral cavity, hair and mucous membranes, are:
- antimicrobial hand washes or hand disinfectants, which are typically rinsed with water after application,
- antimicrobial or disinfecting hand rubs, which are typically leave-on products not rinsed off after application,
- antimicrobial or disinfecting products for skin, oral cavity, hair and mucous membranes for human beings or animals.

Formulation types of the cleaners are selected from the group comprising: cleaner and disinfectant concentrate, liquid cleaners or disinfectants, pulverulent cleaners or disinfectants, sprays for cleaning or disinfection, emulsions and gels.

The inanimate surfaces to be treated are selected from the group comprising: glass, plastics, metal, steel, wood, stone materials, ceramic, cement, coatings, composite materials, textiles (natural fibers such as e.g. cotton, wool, silk and synthetic fibers such as polyester, polyolefins (PE, PP etc.), polyamide, polyurethane, PVC etc.), foam materials and upholstery materials and carpets.

In one variant of the present invention, it is a cleaning composition for hard surfaces. Within the context of the invention, hard surfaces include e.g. tiles, ceramic, glass, glass fibers, metals, steel, aluminum, plastic, wood, stone materials, coatings, composite materials, cement and the like, but do not include textiles.

During the cleaning of hard surfaces, it is often necessary to disinfect a surface. In this application, disinfection is understood as meaning the killing of microorganisms or the reduction in the growth of microorganisms. The treatment of the surfaces can be done in a two-step process with cleaning the surface first, and afterwards disinfecting the cleaned surface, using two different products for cleaning and disinfecting whereby between cleaning and disinfection a rinsing step may optionally be performed. Or the cleaning and disinfection is done in one step with a formulation providing cleaning and disinfecting properties. In both cases, after the disinfection, it may be necessary to rinse the disinfected surface, e.g. with water, to remove unwanted residuals. In other cases, the surface is not rinsed after the disinfection step in order to achieve a long-term antimicrobial activity on the treated surfaces by residual amounts of the imidazolium compound defined above.

The present polyimidazolium compounds may also be used as preservatives for material protection. These preservatives are preferably used in cosmetic and/or pharmaceutical formulations.

Furthermore, the polymers of the invention can also be used for control of microorganisms in technical applications as in-can preservative for household products and technical products.

Typical in-can preservative applications of present imidazolium polymers are technical products such as paints and coatings, aqueous emulsions, latexes, adhesives, inks, synthetic glues, protein-based glues, pigment dispersions, mineral slurries, caulks and ad-hesives, tape joint compounds, disinfectants, cleaners, textile fluids, and systems used therewith.

In addition to the preservation of cosmetics, personal care, household and technical products, the present imidazolium polymer may be used for controlling microorganisms in industrial and water containing systems and processes such as cooling towers, heat exchangers, boiler systems, other industrial process water, ballast water, wastewater treatment systems, reverse osmosis water processing, and in swimming pools, spa facilities, pulp and paper manufacture, metal working fluids, leather manufacture.

The biocides of the invention are useful for controlling microorganisms in aqueous or water-containing systems, such as those present in oil and natural gas applications. Examples of such systems include, but are not limited to, injection and produced water, source water for waterflooding and hydraulic fracturing such as pond water and holding tank water, functional fluids such as drilling muds, workover fluids, hydrotest fluids, packer fluids, oil and gas pipelines or fuel.

In addition, the present biocides may be employed in other areas. The biocides of the invention are suitable for use over a wide temperature range. In a preferred embodiment, the biocides are used in aqueous or water-containing systems at a temperature of 20° C or higher (e.g. within the temperature range 20-90°C). In further embodiments, the temperature of the aqueous or water containing system is 60° C or higher, or is 80° C or higher. The present biocides are also effective in combination with a reducing agent, such as a source of sulfide ions present in the aqueous or water-containing system.

In contrast to an disinfection agent, which destroys the microorganism very fast, a preservative provides an effect over a longer period of time. Preservatives thus show a microbiostatic effect.

The concentration of the preservative according to the present invention in a formulation can vary. Generally, the preseratives of the present invention can be incorporated in a commercial formulation at a concentration of between about 0.01 and about 5 weight percentage (wt-%), based on total formulation. More specifically, for cosmetic or pharmaceutical formulations, the amount of the present mixture need not exceed 1.0 wt-%; however, for industrial or household cleaners, up to 5 wt-% can be employed when desired.

An embodiment of the present invention is a personal care formulation comprising 0.01 to 5 wt-% of at least one polyimidazolium compound, such as a compound of formula (I), and 95 to 99.99 wt-% of at least one cosmetically tolerable adjuvant.

Another embodiment of the present invention is a pharmaceutical formulation comprising
0.01 to 5 wt-% of at least one polyimidazolium compound, e.g. of formula (I), , and 95 to 99.99 wt-% of at least one pharmaceutically tolerable adjuvant.

Another embodiment of the present invention is a household formulation comprising at least one polyimidazolium compound, e.g. of formula (I), and adjuvants used in household formulations.

A soap has, for example, the following composition:

| | |
|---|---|
| 0.01 to 5 wt-% | of at least one compound of formula (I), |
| 0.3 to 1 wt-% | of titanium dioxide, |
| 1 to 10 wt-% | of stearic acid, |
| 0 to 10 wt-% | of at least one auxiliary, and |
| ad 100 % | of a soap base, e.g. the sodium salts of tallow fatty acid and coconut fatty acid or glycerol. |

A shampoo has, for example, the following composition:

| | |
|---|---|
| 0.01 to 5 wt-% | of at least one compound of formula (I), |
| 12.0 wt-% | of sodium laureth-2-sulfate, |
| 4.0 wt-% | of cocamidopropyl betaine, |
| 3.0 wt-% | of NaCl, |
| 0 to 10 wt-% | of at least one auxiliary, and |
| ad 100 wt-% | of water. |

A deodorant has, for example, the following composition:

| | |
|---|---|
| 0.01 to 5 wt-% | of at least one compound of formula (I), |
| 60 wt-% | of ethanol, |
| 0.3 wt-% | of perfume oil, |
| 0 to 10 wt-% | of at least one auxiliary, and |
| ad 100 wt-% | of water. |

All wt-%'s are based on the total weight of the compositions.

The invention relates also to an oral composition, comprising from 0.01 to 15 wt-%, based on the total weight of the composition, of at least one compound of formula (I), and orally tolerable adjuvants.

### Example of an oral composition:

| | |
|---|---|
| 10 wt-% | of sorbitol |
| 10 wt-% | of glycerol |
| 15 wt-% | of ethanol |
| 15 wt-% | of propylene glycol |
| 0.5 wt-% | of sodium lauryl sulfate |
| 0.25 wt-% | of sodium methylcocyl taurate |
| 0.25 wt-% | of polyoxypropylene/polyoxyethylene block copolymer |
| 0.10 wt-% | of peppermint flavouring |
| 0.1 to 0.5 wt-% | of at least one compound of formula (I), and |
| 48.6 wt-% | of water. |

The oral composition according to the invention may be, for example, in the form of a gel, a paste, a cream or an aqueous preparation (mouthwash).

The oral composition according to the invention may also comprise compounds that release fluoride ions which are effective against the formation of caries, for example inorganic fluoride salts, e.g. sodium, potassium, ammonium or calcium fluoride, or organic fluoride salts, e.g. amine fluorides, which are known under the trade name Olafluor.

The present compounds, e.g. those of formula (I), can be used also in household and all-purpose cleaners for cleaning hard surfaces. A cleaning preparation has, for example, the following composition:

| | |
|---|---|
| 0.01 to 5 wt-% | of at least one compound of formula (I), and |
| 3.0 wt-% | octyl alcohol 4EO |
| 1.3 wt-% | fatty alcohol C₈-C₁₀polyglucoside |
| 3.0 wt-% | isopropanol |
| 0 to 5 wt-% | of auxiliaries, |
| ad 100 wt-% | water. |

Also possible, in addition to the preservation of cosmetics, personal care and household products is the use of the inventive biocide in controlling microorganisms in technical products as well as in industrial and water containing systems and processes such as cooling towers, heat exchangers, boiler systems, other industrial process water, ballast water, wastewater treatment systems, reverse osmosis water processing but also swimming pools and spa facilities, pulp and paper manufacture, metal working fluids, leather manufacture, paints and coatings, aqueous emulsions, latexes, adhesives, inks, synthetic and protein-based glues, pigment dispersions, mineral slurries, caulks and adhesives, tape joint compounds, disinfectants, cleaners, textile fluids, or a system used therewith.

Moreover, the biocides of the invention are useful for controlling microorganisms in aqueous or water-containing systems, such as those present in oil and natural gas applications. Examples of such systems include, but are not limited to, injection and produced water, source water for waterflooding and hydraulic fracturing such as pond water and holding tank water, functional fluids such as drilling muds, workover fluids, hydrotest fluids, packer fluids, oil and gas pipelines or fuel.

In addition, the biocides may be employed in other areas. The biocides of the invention are suitable for use over a wide temperature range. In a preferred embodiment, the biocides are used in aqueous or water-containing systems at a temperature of 20° C or greater (e.g. within the temperature range 20-90°C). In further embodiments, the temperature of the aqueous or water containing system is 60° C or greater, or is 80° C or greater. The biocides are also further effective when a reducing agent such as a source of sulfide ion is present in the aqueous or water-containing system.

The formulation of the invention is typically used, for example, as
(a) a home care formulation, such as a disinfectant, all purpose cleaner, dishwashing liquid, descaling agent, a bath room cleaner, a toilet bowl cleaner,
   and/or
(b) a disinfectant and/or sanitary detergent of hard and/or soft surfaces, such as a floor cleaner, a glass cleaner, a kitchen cleaner, a bath room cleaner, a sanitary cleaner, a toilet bowl cleaner, a furniture cleaner,
   and/or
(c) a product for clean in place,
(d) a product for human skin, hair, oral cavity and mucous membanes, such as a disinfecting hand wash, a disinfecting hand rub, a skin lotion, skin cream, skin spray, mouth-wash, toothpaste, or intimate wash liquids, creams, sprays and gels,
e) product for animal uses, such as antiseptic washes, hoof disinfectants, udder disinfectants, dog shampoos,
or for the manufacture of said home care formulation (a), disinfectant and/or sanitary detergent (b), product for clean in place (c), product for human skin, hair, oral cavity and mucous membanes (d), product for animal uses (e).

In a further aspect the present invention is directed to a product comprising the formulation according to this invention, wherein said product is selected from the group consisting of home care formulation, disinfectant of hard and/or soft surfaces, sanitary detergent of hard and/or soft surfaces, and product for clean in place application.

The invention thus further provides a product selected from the group consisting of home care formulation, disinfectant of hard and/or soft surfaces, sanitary detergent of hard and/or soft surfaces, product for clean in place, all purpose cleaner, dishwashing liquid, descaling agent, bath room cleaner, toilet bowl cleaner, floor cleaner, glass cleaner, kitchen cleaner, sanitary cleaner, furniture cleaner, wherein said product comprises a formulation containing the polymeric, ionic compound comprising at least 5 imidazolium groups and containing alkyl moieties as end groups, as described above.

Formulations for certain application fields such as home care, personal care, sanitary disinfection, hand disinfection, and especially for hospital disinfection, are advantageously applied as liquid formulations by means of a suitable dispenser, which allows applying the required dosage of the formulation. Formulations applied in this manner typically comprise the imidazolium polymer as described above (e.g. of the formula I), a liquid carrier (typically water and/or one or more alcohols, where the alcohol may also possess antimicrobial properties), and optionally further components, which are typically selected from surfactants and further antimicrobial agents.

The term "polymer", "polymeric ionic compound", "polyimidazolium compound", "imidazolium polymer", "antimicrobial polymer", and "polymer compound", as used within this specification including the following examples, denotes the polymeric salts of the invention, including those of the formula I.

The following examples illustrate the invention. Unless indicated otherwise, reactions take place at standard conditions, i.e. atmospheric pressure and room temperature (r.t.), which depicts a temperature from the range 20-25°C; over night means a period of 12 to 15 hours; all percentages are given by weight (w/w %), if not indicated otherwise.

Abbreviations:
- Mw: molecular weight (weight average, usually as detected by GPC)
- Mn: molecular weight (number average, usually as detected by GPC)
- M: concentration in moles per liter
- ml: milliliter
- GPC: gel permeation chromatography
- PDI: polydispersity (ratio Mw/Mn)

The molecular weight of the polymeric ionic compounds is determined by gel permeation chromatography (GPC):
A set three modified polyacrylate-copolymer (exclusion range 100 - 30 000 000 g/mol, NOVEMA Max Ultrahigh) columns of 8 mm width and 30 cm length is used. All columns are calibrated using a kit of poly(2-vinylpyridine) standards (620 to 2070000 g/mol; PSS Polymer Standards Service GmbH, Mainz, Germany) and pyridine (79.1 g/mol) as standards. Eluent is water containing 0.1% (w/w) trifluoroacetic acid and 0,1 M NaCl. The temperature of the columns is 35°C, the injected volume 100 microliter, the concentration 1.5 mg/ml and the flow rate 0.5 ml/min. UV detectors (232 nm) and refractive index detectors are used (DRI Agilent 1100 UV GAT-LCD 503).

The weight average molecular weight (Mw), the number average molecular weight (Mn) and the polydispersity PDI (Mw/Mn) of the polymeric ionic compounds are determined. Further, the GPC data reveal the percentage of species with molecular weight below 2000 g/mol in the present polymer product.

Examples: Polymers of the general formula

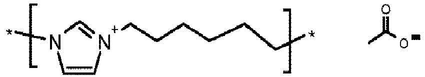

### Example 1, preparation of polymer A:

2 Mol of acetic acid (120.1 g) and 100 g of water are placed in a flask. A mixture of 1.0 mol formaldehyde (30.0 g, as a 49% aq. solution) and 1.0 mol of glyoxal (58.0 g, as a 40% aq. Solution) is added via a dropping funnel to the solution. In parallel, a mixture of 1.0 mol of 1,6-diaminohexane (116 g) in 50 g of water is added to the solution via a separated dropping funnel. During addition of the monomers, the reaction mixture is held at room temperature by ice bath cooling. Addition of both solutions is done over a period of 1 hour. After completion of the addition, the reaction mixture is heated to 100 °C for one further hour. The crude product (containing 39.6% b.w. of Polymer A) is used as received.

| **Analysis** | **Result** |
|---|---|
| Concentration | 39.6 w/w % |
| Mw | 14.300 g/mol |
| Mn | 3.620 g/mol |
| PDI | 4,0 |
| pH | 5.3 |
| Species less than 2000 g/mol | 11.5 w/w % of total polymer |

### Residual Analysis:

| | |
|---|---|
| Glyoxal | 50 mg/kg |
| Formaldehyde | 105 mg/kg |
| Methanol | 0.16 g/100g |
| Formic Acid | 0.14 g/100g |
| Ethanoic Acid | 19.7 g/100g |
| 1,6-Diaminohexane | <0.005 g/100g |

### Example 2, preparation of polymer B (less water as solvent):

The method for preparing polymer A is repeated except that the acetic acid (120.1 g) is provided in a flask without water, and the 1,6-diaminohexane (116 g) in only 20 g of water is added via a separated dropping funnel. The crude product (containing 52,6%) is used as received.

| **Analysis** | **Result** |
|---|---|
| Concentration | 52.6 w/w % |
| Mw | 28.200 g/mol |
| Mn | 4.910 g/mol |
| PDI | 5,7 |
| pH | 5.5 |
| Species less than 2000 g/mol | 6.7 w/w % of total polymer |

### Residual Analysis:

| | |
|---|---|
| Glyoxal | 7 mg/kg |
| Formaldehyde | 313 mg/kg |
| Methanol | 0.15 g/100g |
| Formic Acid | 0.13 g/100g |
| Ethanoic Acid | 25.7 g/100g |
| 1,6-Diaminohexane | 0.01g/100g |

### Example 3, preparation of polymer C (less water as solvent and prolonged addition time of the starting materials):

The method for preparing polymer A is repeated except that 2 Mol of acetic acid (120.1 g) are provided with only 50 g of water in a flask, the 1,6-diaminohexane (116 g) in only 25 g of water is added via separated dropping funnel, and the addition of both monomer solutions is done over a period of 4 hours. The crude product (containing 40,8%) is used as received.

| **Analysis** | **Result** |
|---|---|
| Concentration | 40,8 w/w % |
| Mw | 42300 g/mol |
| Mn | 5900 g/mol |
| PDI | 7,2 |
| pH | 5.4 |
| Species less than 2000 g/mol | 5.5 w/w % of total polymer |

### Residual Analysis:

| | |
|---|---|
| Glyoxal | 143 mg/kg |
| Formaldehyde | 283 mg/kg |
| Methanol | 0.17 g/100g |
| Formic Acid | 0.12 g/100g |
| Ethanoic Acid | 23.6 g/100g |
| 1,6-Diaminohexane | 0.005 g/100g |

### Example 4, preparation of polymer D (less water as solvent, dialysis):

The method for preparing polymer B is repeated. The crude product (containing 52,6%) is subjected to ultrafiltration: The crude product is diluted to a concentration of 10.5 %. Approximately 100 grams of the diluted solution are filled into a stirred test cell fitted with a UP 010 membrane (Microdyn Nadir, 10 kD molecular weight cut-off). The test cell is kept at a temperature of 30 °C and pressurized with nitrogen up to a pressure of 10 bar.

By pressurizing the cell, the solvent and lower-molecular species get pressed through the membrane into the permeate. Larger species are retained by the membrane and stay inside the test cell. In the particular experiment, the setup is operated in a way that each volume of permeate produced is replaced by an equal volume of an acetic acid solution of pH 5.5. The experiment is run until a diafiltration factor of 10 is reached (i.e., an amount of permeate equal to 10x the feed volume is produced).

| **Analysis** | Result |
|---|---|
| Concentration | 19,1 w/w % |
| Mw | 43,700 g/mol |
| Mn | 15700 g/mol |
| PDI | 2,8 |
| pH | 8,0 |
| Species less than 2000 g/mol | 0.7 w/w % of total polymer |

### Residual Analysis:

| | |
|---|---|
| Glyoxal | 1 mg/kg |
| Formaldehyde | 41 mg/kg |
| Methanol | < 100 mg/kg |
| Formic Acid | 0.05 g/100g |
| Ethanoic Acid | 5.4 g/100g |
| 1,6-Diaminohexane | < 0.005 g/100g |

### Analysis summary:

| Polymer | Mw | Mn | PDI | % < 2000 |
|---|---|---|---|---|
| A | 14300 | 3620 | 4.0 | 11.5 |
| B | 28200 | 4910 | 5.7 | 6.7 |
| C | 42300 | 5900 | 7.2 | 5.5 |
| D | 43700 | 15700 | 2.8 | 0.7 |

### Comparative examples: Polymers of WO 2012/127009

Formaldehyde, glyoxal and hexamethylene diamine are reacted with protic acid in the absence of an additional solvent to obtain the comparative polymers identified in the following table (GPC analysis as described above).

| Polymer | C1 (example 11) | C2 (example 12) |
|---|---|---|
| protic acid | CH₃SOOH | CH₃O-[CH₂CH₂O]₂-CH₂COOH |
| Mw | 6060 | 36500 |
| Mn | 970 | 2350 |
| PDI | 6.3 | 15.5 |
| Species <2000g/mol | 47.8 % b.w. | 20.08 % b.w. |

For biological activity testing, the following microorganisms are used:
Staphylococcus aureus (S. aureus ATCC 6538);
Enterococcus hirae
Escherichia Coli (E. coli DSM 682);
Pseudomonas aeruginosa (P. aeruginosa ATCC 15442);
Candida albicans (C. albicans DSM 1386 or ATCC 10231, as indicated);
Aspergillus brasiliensis (A. brasiliensis DSM 1988);
Aspergillus niger
Klebsiella pneumoniae (DSM 789).

### Example 5: Evaluation of Minimial Inhibitory Concentration (MIC)

The polymer of the invention (polyimidazolium salt) is dissolved in water and added to Müller-Hinton-Broth (for bacteria) or Malt extract broth (for fungi) at a dilution of 1:50. After inoculation with the microorganism, samples are incubated at 37°C (in case of bacteria) or 30°C (in case of fungi) for 48 h and evaluated with regard to turbidity of the broth medium. Additionally, aliquots of each sample are cultivated on solid agar media to differentiate between turbidity caused by microbial growth and turbidity caused by sample reactions. Results are documented as Minimal Inhibitory Concentration (MIC) in ppm, i.e. the lowest concentration of the present polyimidazolium compound, which does not allow any microbial growth. The results are compiled in the below table.

**Table: Minimial Inhibitory Concentration (MIC)**

| **Polymer Sample** | **MIC [ppm]** | | | | | |
|---|---|---|---|---|---|---|
| | **S. *aureus*** | ***E. hirae*** | ***E. coli*** | ***P*. *aeruginosa*** | ***C. albicans*** | ***A. niger*** |
| **A** | 3.91 | 3.91 | 3.91 | 3.91 | 7.81 | 62.5 |
| **B** | 7.81 | 1.95 | 3.91 | 3.91 | 15.63 | 62.5 |
| **C** | 7.81 | 1.95 | 3.91 | 3.91 | 15.63 | 31.25 |
| **D** | 7.81 | 1.95 | 3.91 | 3.91 | 15.63 | 31.25 |

### Example 6: Evaluation of bactericidal activity in solution

The polymer of the invention is dissolved in water and tested according to European Standard DIN EN 1276-2010 in standardized hard water with additional soiling of 0.3% bovine albumin. The final test composition contains the polymer of the invention in a concentration of 12.5 ppm (weight parts per million). Results after contact time of 5 min and for 1 minute, respectively, are documented in the following table as logarithmic reduction (Ig R) in comparison to the number of microorganism used for the test.

**Table: Bactericidal activity in solution (DIN EN 1276-2010) expressed as logarithmic reduction**

| Polymer Sample | | [Ig R] | | | |
|---|---|---|---|---|---|
| | | *S*. *aureus 5 min* | *S. aureus 1 min* | *P. aeruginosa 5 min* | *P. aeruginosa 1 min* |
| A | | > 5.0 | 2.3 | > 5.1 | 4.8 |
| B | | 4.8 | 3.2 | > 5.1 | 4.9 |
| C | | 4.9 | 3.8 | > 5.5 | 5.0 |
| D | | | | >5.5 | 5.2 |

### Example 7: Evaluation of bactericidal activity in formulation

The polymer of the invention is dissolved in standardized hard water (DIN EN 1276-2010), then further components such as nonionic surfactant and chelating agent is added, finally pH is adjusted to 8.0. The complete formulation, representing an All-purpose-cleaner with a high amount of nonionic surfactant, is shown in the table below. For the purpose of comparison, a further formulation is prepared, whose composition is identical, except that the quantity of polymer of the invention is replaced by water.

| **Ingredients** | **Test Formulation [% w/w]** |
|---|---|
| Glucopon® 215 UP (Alkylpolyglucosid, 64%) | 3.9 |
| Citric acid | 0.8 |
| Sodium bicarbonate | 0.8 |
| Trilon® M powder (MGDA) | 0.3 |
| **Biocide** | **0.06 - 0.08** (resulting in 0.03% b.w. active polymer) |
| Water | add to 100 |

Antimicrobial testing of the above formulation (pH 8) is done according to European Standard (DIN EN 1276-2010) under dirty conditions, i.e. additional soiling of 0.3% bovine albumin, as 80%, i.e. the final test composition contains the polymer of the invention in a concentration of 0.024% Results are documented in the table below as logarithmic reduction (Ig R) in comparison to the number of microorganism used for the test.

| |
|---|
| DIN EN 1276-2010, test product containing 0.03% of active polymer contact time 5 min; dirty conditions (0.3% bovine albumin) |
| [results as log reduction R] |

| Biocide | *S*. *aureus* | *P. aeruginosa* |
|---|---|---|
| | [Ig R] | [Ig R] |
| Polymer A | > 5.1 | > 5.2 |
| Polymer B | > 5.1 | > 5.2 |
| Polymer C | > 5.2 | > 5.4 |
| Reference (without biocide) | < 1.1 | < 1.3 |

### Example 8: Skin Sensitization Test

A skin sensitization test using a 3D reconstructed human epidermidis (EpiSkin®; Episkin, Lyon, France) is carried out in accordance with the SENS-IS test protocol for the in vitro detection of sensitizers (F. Cottrez et al., Toxicol. in Vitro 32, 248 (2016)). The SENS-IS assay is based on the quantitative analysis of specific gene biomarkers. A substance is classified as negative or non-sensitizer in case the number of overex-pressed genes is less than 7 in both gene groups involved in skin sensitization. Results are shown in the following tables.

### Test using Polymer C:

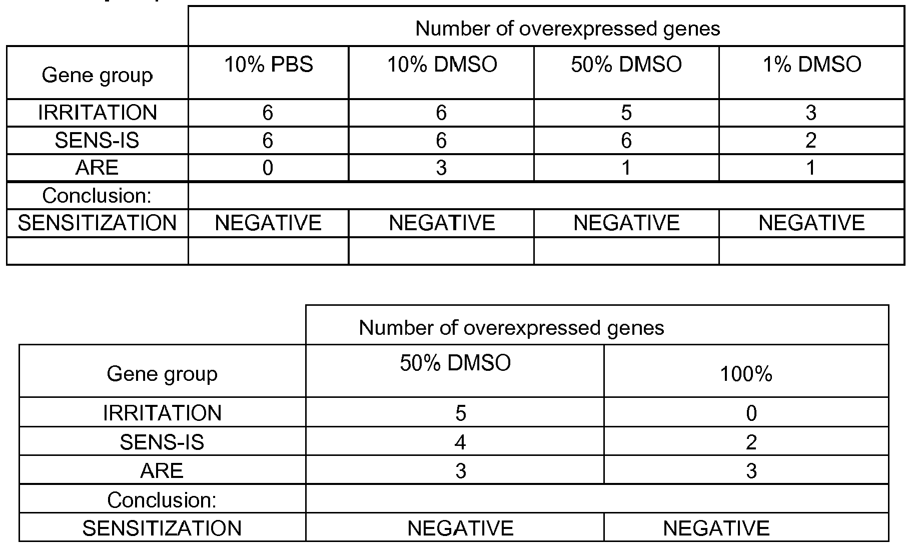

### Test using Polymer A:

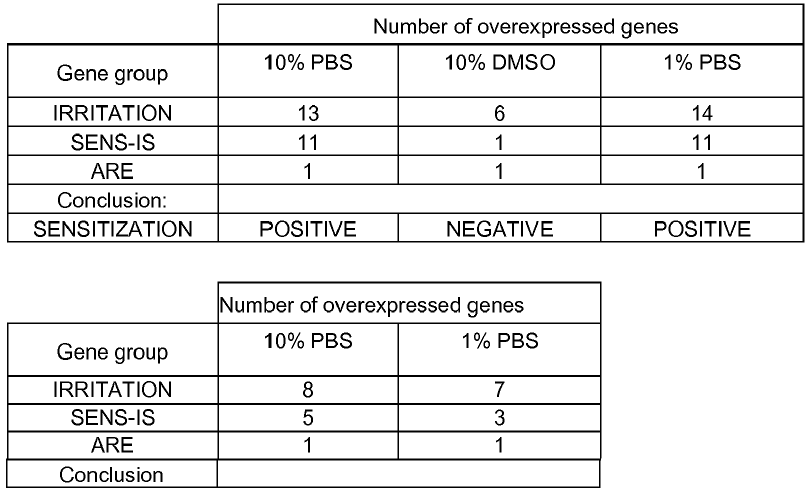

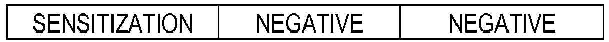

The results of the study with Polymer C show a clear negative result, based on which Polymer C can be classified as non-sensitizer.

## Claims

1. Antimicrobial composition comprising at least one carrier and/or at least one auxiliary agent, and as an antimicrobial agent at least one polymeric, ionic compound containing alkylene linked imidazolium groups,
which polymeric, ionic compound is obtainable by reacting glyoxal, formaldehyde, at least one C₄-C₈alkylene diamine and at least one protic acid, and is **characterized in that** its number average molecular weight is from the range 2500 to 19000 g/mol, the amount of species having a molar mass below 2000 g/mol is less than 12 % of the total weight of the polymeric ionic compound, and its polydispersity is from the range 2 to 9, as determined by gel permeation chromatography, especially using modified polyacrylate-copolymer gel, poly(2-vinylpyridine) standards, and water containing 0.1% (w/w) trifluoroacetic acid and 0,1 mol of sodium chloride per liter as eluent at 35°C.

2. The composition of claim 1, wherein the C₄-C₈alkylene diamine is hexamethylene diamine, and wherein the reaction mixture for obtaining the polymeric, ionic compound is essentially free of monoamines.

3. The composition of claim 1 or 2, wherein the polymeric, ionic compound is **characterized by** a number average molecular weight from the range 3000 to 19000, and especially has a weight average molecular weight from the range 20000 to 80000 and/or the amount of species having a molar mass below 2000 g/mol is less than 8 % of the total weight of the polymeric ionic compound.

4. The composition of claim 1 or 2 or 3, wherein the protic acid in the reaction mixture for obtaining the polymeric, ionic compound is selected from C₁-C₂₀carboxylic acids and mineralic acids; such as formic acid, hydrochloric acid, hydrobromic acid, acetic acid, aliphatic carboxylic acids having 3 to 20 carbon atoms, H₂SO₄, HNO₃, H3PO4, C₁-C₈sulfonic acids, trifluormethyl-sulfonic acid; especially acetic acid.

5. The composition according to any of claims 1 to 4, wherein the polymeric, ionic compound comprises a polymer of the formula I 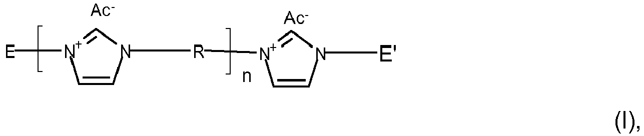 wherein
E is C₄-C₈ aminoalkyl or C₄-C₈ ammoniumalkyl, whose alkyl moiety preferably is straight chain alkyl carrying a terminal amino or ammonium group;
E' is as defined for E, or is hydrogen;
R is C₄-C₈alkylene, preferably of straight chain;
Ac- is the anion of the protonic acid used according to method described in claim 1, especially an anion of a protic acid noted in claim 3, for example an anion selected from HCOO, CH3COO, C₃-C₁₉ alkylcarboxylate, HSO₄, sulphate, nitrate, H₂PO₄, HPO₄, PO₄, C₁-C₈sulfonate, trifluormethyl-sulfonate, or halogenides such as chloride, bromide iodide; especially acetate; and
n denotes the number of repeating units to obtain a number average molecular weight as defined in claim 1 or claim 3;
preferably, n ranges from 10 to 75, most preferably from 15 to 60.

6. The composition according to claim 5, wherein the polymeric, ionic compound comprises a polymer of the formula I, wherein E is aminohexyl or ammoniumhexyl, R is hexylene, and Ac⁻ stands for the acetyl anion.

7. The composition according to any of claims 1 to 6 comprising a liquid carrier and preferably a surfactant, wherein the liquid carrier is preferably selected from water, water miscible solvents and mixtures of water and water miscible solvents.

8. The composition according to any of claims 1 to 7 comprising an auxiliary agent selected from the group consisting of
- cationic surfactants, such as quaternized ethoxylated fatty oils, quaternized ethoxylated fatty amines;
- non cationic surfactants, preferably selected from quaternized ethoxylated fatty oils or quaternized ethoxylated fatty amines and non-ionic surfactants, such as polyethers or condensated products of ethylene oxide and/or propylene oxide with fatty acids, fatty alcohols, fatty amides, polyhydric alcohols; especially alcohol ethoxylates, alkylated polyglycosides, amine ethoxylates, polyethers;
- thickeners and binders, such as polyethylene imines, cationic polyacrylates, cationic polyacrylamides, polyvinylpyrrolidones, and nonionic or amphoteric polymers;
- chelating agents like polycarboxylic acids and salts thereof, such as ethylenediaminetetraacetic acid, its di-, tri- or tetrasodium salt, the trisodium salt of methylglycinediacetic acid, nitrilotriacetic acid and salts thereof;
- further antimicrobial agents, especially aldehydes, alcohols, chlorinated hydroxydiphenylethers, such as glutaraldehyde, glyoxal, phenoxyethanol, diclosan

9. The composition according to any of claims 1 to 8 comprising a surfactant as an auxiliary agent selected from the group consisting of cationic surfactants and non-ionic surfactants, and preferably selected from quaternized ethoxylated fatty oils and quaternized ethoxylated fatty amines as cationic surfactants, and from polyethers and condensated products of ethylene oxide and/or propylene oxide with fatty acids, fatty alcohols, fatty amides, polyhydric alcohols; especially from alcohol ethoxylates, alkylated polyglycosides, amine ethoxylates, polyethers as non-ionic surfactants.

10. The composition according to any of claims 1 to 9 comprising a thickener or binder as an auxiliary agent, which is preferably selected from polyethylene imines, cationic polyacrylates, cationic polyacrylamides, polyvinylpyrrolidones, and nonionic or amphoteric polymers.

11. The composition according to any of claims 1 to 10 comprising a chelating agent as an auxiliary agent, which is preferably selected from polycarboxylic acids and salts thereof; especially ethylenediaminetetraacetic acid, the disodium salt of ethylenediaminetetraacetic acid, the trisodium salt of ethylenediaminetetraacetic acid, the tetrasodium salt of ethylenediaminetetraacetic acid, the trisodium salt of methylglycinediacetic acid, nitrilotriacetic acid, the monosodium salt of nitrilotriacetic acid, the disodium salt of nitrilotriacetic acid, the trisodium salt of nitrilotriacetic acid.

12. The composition according to any of claims 1 to 11 comprising a further antimicrobial agent as an auxiliary agent, which is preferably selected from aldehydes, alcohols, chlorinated hydroxydiphenylethers, especially glutaraldehyde, glyoxal, phenoxyethanol, 4,4'-dichloro 2-hydroxy diphenylether (also known as diclosan), 2,4,4'-trichloro 2'-hydroxy diphenylether (also known as triclosan).

13. The composition as claimed in any of claims 1 to 12, wherein the composition is
- a personal care composition, or
- a home care composition, or
- a composition used for industrial or institutional or hospital disinfection, or
- a composition for industrial and water containing systems and processes, or
- a material protection composition, or
- an in-can preservation composition, or
- a composition used for microbial control of industrial processes such as pulp and paper making, water treatment, oil and gas exploration, or
- a pharmaceutical composition.

14. The composition as claimed in any of claims 1 to 13 containing, on 100 % total weight of the composition, the polymeric, ionic compound in an amount ranging from 0.0001 to 30 % by weight of the composition, and the carrier and/or auxiliary agent in an amount ranging from 5 to 99.9999 % by weight; especially containing an auxiliary agent and a liquid carrier in accordance with claim 7, where the auxiliary agent is selected in accordance with any of claims 8 to 12 and is present in an amount ranging from 0.0001 to 30 % by weight of the composition.

15. A method for combating harmful organisms or for protecting human beings, animals, materials or processes from the effects of these harmful organisms, wherein the habitat of the harmful organism or the human being, animal or material to be protected is brought into contact with a biocide composition or the biocide composition as defined in any of the preceding claims; wherein the harmful organisms especially are bacteria, yeasts, moulds, fungi and/or viruses; which method is not a method for treatment of the human or animal body by surgery or therapy or diagnostic method practised on the human or animal body.

16. The use of at least one polymeric, ionic compound comprising imidazolium groups as defined in any of claims 1 to 6 as a biocide.

17. Process for the preparation of a polymeric, ionic compound comprising imidazolium groups as described in any of claims 1 to 5, which process comprises simultaneously reacting glyoxal, formaldehyde, at least one C₄-C₈alkylene diamine, and at least one protic acid, in the absence of a monoamine, and in the presence of 80 to 500 ml of a polar solvent on 1 mol of alkylene diamine in the reaction mixture.

18. Process of claim 17, wherein the reaction product is subsequently subjected to ultrafiltration, for example using a membrane with cut-off between 5 and 15 kD molecular weight.
